# EUROPEAN PATENT APPLICATION

(11) **EP 4 382 539 A1**
(43) Date of publication of application: **12.06.2024**
(21) Application number: 22853038.2
(22) Date of filing: 02.08.2022
(51) Int. Cl.: C07K 16/42, A61K 39/395, A61P 35/00, A61P 37/06, C12N 15/13, C12P 21/08, C12Q 1/02, G01N 33/53, C07K 19/00, C12N 5/10

(54) **ANTIBODY BINDING TO LINKER OF SCFV OR LIKE**

(30) Priority: 02.08.2021 JP 2021126997; 14.04.2022 JP 2022066687
(71) Applicant: Noile-Immune Biotech Inc., Tokyo 105-0012 (JP)
(72) Inventor: TAMADA Koji, Ube-shi, Yamaguchi 755-8505 (JP); SAKODA Yukimi, Ube-shi, Yamaguchi 755-8505 (JP); ADACHI Keishi, Ube-shi, Yamaguchi 755-8505 (JP)
(74) Representative: Hoffmann Eitle
(86) International application number: PCT/JP2022/029582
(87) International publication number: WO 2023/013619

(57) **Abstract**

The present invention provides an antibody that binds to a linker in a new style of binding. More specifically, the present invention provides an antibody that binds to the linking part between a first region and a linker and the linking part between a linker and a second region in an scFv structure.

## Description

### Technical Field

The present invention relates to an antibody that binds to a linker of, e.g., an scFv. The present invention particularly relates to an antibody that binds to a linking part between the framework region of an scFv and a linker region and capable of recognizing a wide variety of scFvs regardless of the sequences of CDR regions of the scFv.

### Background Art

In a fusion protein, proteins are sometimes linked via a linker. As the linker, a flexible linker such as a GS linker consisting of G and S is used and is required for some uses to avoid structural restriction in linking the proteins.

As an antibody fragment, an scFv is known. In the scFv, a heavy-chain variable region and a light-chain variable region of an antibody is linked via a linker. Such a fusion protein can be detected by protein L. However, protein L has an affinity for other immunoglobulins and the specificity of protein L to an scFv is not high. Because of this, protein L is not suitably used in *in vivo* or a system where other immunoglobulins are present (Non Patent Literature 1). An antibody that binds to the linker of such a fusion protein has been developed. For example, Patent Literature 1 discloses an antibody that binds to Whitlow linker. These literatures disclose that an amino acid such as proline (P), lysine (K) or aspartic acid (D) is inserted into the linker to overcome the low immunogenicity of the linker.

### Citation List

### Patent Literature

Patent Literature 1: US2019/0092818A

### Non Patent Literature

Non Patent Literature 1: Hu Y. and Huang J., Front. Immunol. 11: 1770, 2020.

### Summary of Invention

The present invention provides an antibody that binds to a linker of, e.g., an scFv. More specifically, the present invention provides an antibody that binds to a linking part between a heavy-chain variable region or a light-chain variable region of an antibody and a linker in an scFv.

The present inventors prepared an antibody that binds to the linking part of a heavy-chain variable region or a light-chain variable region of an antibody and a linker. As a result, they have found that an antibody that binds to a peripheral portion of a linker can be obtained regardless of the level of immunogenicity of the linker itself. The antibody obtained was found to bind to a wide variety of scFvs regardless of the sequences of complementarity-determining regions (CDRs) of the scFv. The present invention was attained based on the findings.

According to the present invention, for example, the following inventions are provided.
[1] An antibody that binds to an scFv containing a first region, a linker, and a second region, wherein the first region, the linker, and the second region in the scFv are arranged in this order in the direction from its N terminal to its C terminal,
   the first region and second region are a heavy-chain variable region of an antibody and a light-chain variable region of an antibody, respectively or a light-chain variable region of an antibody and a heavy-chain variable region of an antibody, respectively, and
   the antibody is an antibody satisfying at least one of the following (i) to (vi):
      (i) the antibody binds to an isolated peptide having an amino acid sequence of (i-a) a region present within the first region and having 1 to 10 amino acids from the C terminal of the first region, and (i-b) a partial region of a linker continuous with the region and having 1 to 10 amino acids from the N terminal of the linker, the isolated peptide having 13 to 20 amino acid length,
      (ii) the antibody binds to an isolated peptide having an amino acid sequence of (ii-a) a partial region of a linker continuous with the second region and having 1 to 10 amino acids from the C terminal of the linker, and (ii-b) a region present within the second region and having 1 to 10 amino acids from the N terminal of the second region, the isolated peptide having 13 to 20 amino acid length,
      (iii) the antibody binds to an isolated peptide having an amino acid sequence set forth in any one of SEQ ID NOs: 19, 20, 23 and 24,
      (iv) the antibody binds to an isolated peptide having an amino acid sequence set forth in any one of SEQ ID NOs: 19 to 24 and 50 to 71,
      (v) the antibody binds to (v-a) a region present within the first region and having 1 to 10 amino acids from the C terminal of the first region, and/or (v-b) a partial region of a linker continuous with the region and having 1 to 10 amino acids from the N terminal of the linker (for example, a linking part between the first region and the linker), and
      (vi) the antibody binds to (vi-a) a partial region of a linker continuous with the second region and having 1 to 10 amino acids from the C terminal of the linker, and/or (vi-b) a region present in the second region and having 1 to 10 amino acids from the N terminal of the second region (for example, a linking part between the second region and the linker).
[2] The antibody according to item [1], wherein (i) the antibody binds to an isolated peptide having an amino acid sequence of (i-a) a region present within the first region and having 1 to 10 amino acids from the C terminal of the first region, and (i-b) a partial region of a linker continuous with the region and having 1 to 10 amino acids from the N terminal of the linker, the isolated peptide having a 13 to 20 amino acid length.
[3] The antibody according to item [1], wherein (ii) the antibody binds to an isolated peptide having an amino acid sequence of (ii-a) a partial region of a linker continuous with the second region and having 1 to 10 amino acids from the C terminal of the linker, and (ii-b) a region present within the second region and having 1 to 10 amino acids from the N terminal of the second region, the isolated peptide having a 13 to 20 amino acid length.
[4] The antibody according to item [1], wherein (iii) the antibody binds to an isolated peptide having an amino acid sequence set forth in any one of SEQ ID NOs: 19, 20, 23 and 24.
[5] The antibody according to item [1], wherein (iv) the antibody binds to an isolated peptide having an amino acid sequence set forth in any one of SEQ ID NOs: 19 to 24 and 50 to 71.
[6] The antibody according to item [1], wherein (v) the antibody binds to (v-a) a region present within the first region and having 1 to 10 amino acids from the C terminal of the first region, and/or (v-b) a partial region of a linker continuous with the region and having 1 to 10 amino acids from the N terminal of the linker (for example, a linking part between the first region and the linker).
[7] The antibody according to item [1], wherein (vi) the antibody binds to (vi-a) a partial region of a linker continuous with the second region and having 1 to 10 amino acids from the C terminal of the linker, and/or (vi-b) a region present in the second region and having 1 to 10 amino acids from the N terminal of the second region (for example, a linking part between the second region and the linker).
[8] The antibody according to any one of items [1] to [7], wherein (vii) the antibody exhibits no significant binding affinity for an scFv in which the order of the heavy-chain variable region and the light-chain variable region is interchanged.
[9] The antibody according to any one of items [1] to [7], wherein (viii) the antibody exhibits significant binding affinity even for an scFv in which the order of the heavy-chain variable region and the light-chain variable region is interchanged.
[10] The antibody according to any one of items [1] to [9], wherein the antibody binds to a chimeric antigen receptor (CAR) containing the scFv.
[11] The antibody according to any one of items [1] to [10], wherein the antibody binds to a cell expressing the chimeric antigen receptor (CAR) containing the scFv.
[12] A method for detecting a cell expressing a chimeric antigen receptor (CAR) containing an scFv, including bringing the cell into contact with the antibody according to item [11].
[13] A kit or test agent for detecting a cell expressing a chimeric antigen receptor (CAR) containing an scFv, wherein the kit or test agent contains the antibody according to item [11].
[14] A method for targeting a cell expressing a chimeric antigen receptor (CAR) containing an scFv in a subject, including
   administering the cell expressing the chimeric antigen receptor (CAR) containing an scFv to the subject, and
   administering the antibody according to item [11] to the subject.
[15] An antibody formulation for use in targeting a cell expressing a chimeric antigen receptor (CAR) containing an scFv in a subject administered with the cell expressing a chimeric antigen receptor (CAR) containing an scFv, wherein the antibody formulation contains the antibody according to item [11].
[16] A method for obtaining (or a method for selecting or identifying) an antibody that binds to an scFv, wherein
   the scFv contains a first region, a linker, and a second region, wherein the first region, the linker, and the second region in the scFv are arranged in this order in the direction from the N terminal to the C terminal; and the first region and second region are a heavy-chain variable region of an antibody and a light-chain variable region of an antibody, respectively, or a light-chain variable region of an antibody and a heavy-chain variable region of an antibody, respectively,
   the method including selecting or identifying, from a group of antibodies, an antibody that binds to an scFv and satisfying at least one of (i) to (vi):
      (i) the antibody binds to an isolated peptide having an amino acid sequence of a region present within the first region and having 1 to 10 amino acids from the C terminal of the first region, and a partial region of a linker continuous with the region and having 1 to 10 amino acids from the N terminal of the linker, the isolated peptide having 13 to 20 amino acid length;
      (ii) the antibody binds to an isolated peptide having an amino acid sequence of a partial region of a linker continuous with the second region and having 1 to 10 amino acids from the C terminal of the linker, and a region present within the second region and having 1 to 10 amino acids from the N terminal of the second region, the isolated peptide having 13 to 20 amino acid length;
      (iii) the antibody binds to an isolated peptide having an amino acid sequence set forth in any one of SEQ ID NOs: 19, 20, 23 and 24;
      (iv) the antibody binds to an isolated peptide having an amino acid sequence set forth in any one of SEQ ID NOs: 19 to 24 and 50 to 71;
      (v) the antibody binds to a region present within the first region and having 1 to 10 amino acids from the C terminal of the first region and a partial region of a linker continuous with the region and having 1 to 10 amino acids from the N terminal of the linker; and
      (vi) the antibody binds to a partial region of a linker continuous with the second region and having 1 to 10 amino acids from the C terminal of the linker, and a region present within the second region and having 1 to 10 amino acids from the N terminal of the second region.
[17] The method according to item [16], wherein (vii) the antibody exhibits no significant binding affinity for an scFv in which the order of the heavy-chain variable region and the light-chain variable region is interchanged.
[18] The method according to item [16], wherein (viii) the antibody exhibits significant binding affinity even for an scFv in which the order of the heavy-chain variable region and the light-chain variable region is interchanged.
[19] The method according to any one of items [16] to [18], further including allowing an antibody-producing cell to produce an antibody containing amino acid sequences of heavy-chain CDR1 to 3 and light-chain CDR1 to 3 of the antibody selected.
[20] The antibody according to any one of items [2] to [7], further satisfying (vii).
[21] The antibody according to any one of items [2] to [7], further satisfying (viii).
[22] The antibody according to any one of items [2] to [8], wherein the antibody does not bind, or binds only with low binding affinity, to a peptide not containing a partial peptide of the first region or second region and containing the amino acid sequence of a linker portion.
[23] The antibody according to any one of items [2] to [7] and [9], wherein the antibody binds even to a peptide not containing a partial peptide of the first region or second region and containing the amino acid sequence of a linker portion.
[24] The antibody according to item [10] or [11], further satisfying (vii).
[25] The antibody according to item [10] or [11], further satisfying (viii).
[26] The antibody according to item [10] or [11], wherein the antibody does not bind, or binds only with low binding affinity, to a peptide not containing a partial peptide of the first region or second region and containing the amino acid sequence of a linker portion.
[27] The antibody according to item [10] or [11], wherein the antibody binds even to a peptide not containing a partial peptide of the first region or second region and containing the amino acid sequence of a linker portion.
[28] The antibody according to any one of items [20] to [27], wherein the antibody binds to a chimeric antigen receptor (CAR) containing an scFv.
[29] A composition containing the antibody according to any one of items [20] to [28].
[30] A method for detecting a cell expressing the chimeric antigen receptor (CAR) containing an scFv, including bringing the cell into contact with the antibody according to item [28].
[31] A kit or test agent for detecting a cell expressing the chimeric antigen receptor (CAR) containing an scFv, wherein the kit or test agent contains the antibody according to item [28].
[32] A method for targeting a cell expressing the chimeric antigen receptor (CAR) containing an scFv in a subject, including
   administering the cell expressing the chimeric antigen receptor (CAR) containing an scFv to the subject, and
   administering the antibody according to item [28] to the subject.
[33] An antibody formulation for use in targeting a cell expressing the chimeric antigen receptor (CAR) containing an scFv in a subject administered with the cell expressing the chimeric antigen receptor (CAR) containing an scFv, wherein the antibody formulation contains the antibody according to item [28].
[34] The antibody according to item [1], wherein the group of antibodies can be obtained by immunizing a non-human mammal with an isolated short-chain peptide as defined by any one of item [1] (i) to (iv).
[35] The antibody according to item [1] (i), wherein the group of antibodies can be obtained by immunizing a non-human mammal with an isolated short-chain peptide that can be defined by item [1] (i).
[36] The antibody according to item [1] (ii), wherein the group of antibodies can be obtained by immunizing a non-human mammal with an isolated short-chain peptide that can be defined by item [1] (ii).
[37] The antibody according to item [1] (iii), wherein the group of antibodies can be obtained by immunizing a non-human mammal with an isolated short-chain peptide that can be defined by item [1] (iii).
[38] The antibody according to item [1] (iv), wherein the group of antibodies can be obtained by immunizing a non-human mammal with an isolated short-chain peptide that can be defined by item [1] (iv).
[39] The method according to any one of items [16] to [18], wherein the group of antibodies can be obtained by immunizing a non-human mammal with an isolated short-chain peptide that can be defined by item [16] (i), and the method includes selecting an antibody according to [16] (i).
[40] The method according to item [16], wherein the group of antibodies can be obtained by immunizing a non-human mammal with an isolated short-chain peptide that can be defined by item [16] (ii), and the method includes selecting an antibody according to item [16] (ii).
[41] The method according to item [16], wherein the group of antibodies can be obtained by immunizing a non-human mammal with an isolated short-chain peptide that can be defined by item [16] (iii), and the method includes selecting an antibody according to item [16] (iii) .
[42] The method according to item [16], wherein the group of antibodies can be obtained by immunizing a non-human mammal with an isolated short-chain peptide that can be defined by item [16] (iv), and the method includes selecting an antibody according to item [16] (iv).
[43] The method according to item [17], wherein the group of antibodies can be obtained by immunizing a non-human mammal with an isolated short-chain peptide that can be defined by item [16] (i), and the method includes selecting an antibody according to item [16] (i), which does not exhibit significant binding affinity for an scFv in which the order of the heavy-chain variable region and the light-chain variable region is interchanged.
[44] The method according to item [17], wherein the group of antibodies can be obtained by immunizing a non-human mammal with an isolated short-chain peptide that can be defined by item [16] (ii), and the method includes selecting an antibody according to item [16] (ii), which does not exhibit significant binding affinity for an scFv in which the order of the heavy-chain variable region and the light-chain variable region is interchanged.
[45] The method according to item [17], wherein the group of antibodies can be obtained by immunizing a non-human mammal with an isolated short-chain peptide that can be defined by item [16] (iii), and the method includes selecting an antibody according to item [16] (iii), which does not exhibiting significant binding affinity for an scFv in which the order of the heavy-chain variable region and the light-chain variable region is interchanged.
[46] The method according to item [17], wherein the group of antibodies can be obtained by immunizing a non-human mammal with an isolated short-chain peptide that can be defined by item [16] (iv), and the method includes selecting an antibody according to item [16] (iv), which does not exhibit significant binding affinity for an scFv in which the order of the heavy-chain variable region and the light-chain variable region is interchanged.
[47] The method according to item [18], wherein the group of antibodies can be obtained by immunizing a non-human mammal with an isolated short-chain peptide that can be defined by item [16] (i), and the method includes selecting an antibody according to item [16] (i), which exhibits significant binding affinity even for an scFv in which the order of the heavy-chain variable region and the light-chain variable region is interchanged.
[48] The method according to item [18],wherein the group of antibodies can be obtained by immunizing a non-human mammal with an isolated short-chain peptide that can be defined by item [16] (ii), and the method includes selecting an antibody according to item [16] (ii), which exhibits significant binding affinity even for an scFv in which the order of the heavy-chain variable region and the light-chain variable region is interchanged.
[49] The method according to item [18],wherein the group of antibodies can be obtained by immunizing a non-human mammal with an isolated short-chain peptide that can be defined by item [16] (iii), and the method includes selecting an antibody according to item [16] (iii), which exhibits significant binding affinity even for an scFv in which the order of the heavy-chain variable region and the light-chain variable region is interchanged.
[50] The method according to item [18], wherein the group of antibodies can be obtained by immunizing a non-human mammal with an isolated short-chain peptide that can be defined by item [16] (iv), and the method includes selecting an antibody according to item [16] (iv), which exhibits significant binding affinity even for an scFv in which the order of the heavy-chain variable region and the light-chain variable region is interchanged.
[51] An antibody that binds to an scFv, containing: a heavy-chain variable region that contains heavy-chain CDR1 having an amino acid sequence set forth in SEQ ID NO: 92, heavy-chain CDR2 having an amino acid sequence set forth in SEQ ID NO: 93, and heavy-chain CDR3 having an amino acid sequence set forth in SEQ ID NO: 94; and a light-chain variable region that contains light-chain CDR1 having an amino acid sequence set forth in SEQ ID NO: 96, light-chain CDR2 having an amino acid sequence set forth in SEQ ID NO: 97, and light-chain CDR3 having an amino acid sequence set forth in SEQ ID NO: 98.
[52] An antibody that binds to an scFv, containing: a heavy-chain variable region that contains heavy-chain CDR1 having an amino acid sequence set forth in SEQ ID NO: 100, heavy-chain CDR2 having an amino acid sequence set forth in SEQ ID NO: 101, and heavy-chain CDR3 having an amino acid sequence set forth in SEQ ID NO: 102; and a light-chain variable region that contains light-chain CDR1 having an amino acid sequence set forth in SEQ ID NO: 104, light-chain CDR2 having an amino acid sequence set forth in SEQ ID NO: 105, and light-chain CDR3 having an amino acid sequence set forth in SEQ ID NO: 106.
[53] The antibody according to item [51], containing a heavy-chain variable region having an amino acid sequence set forth in SEQ ID NO: 91 and a light-chain variable region having an amino acid sequence set forth in SEQ ID NO: 95.
[54] The antibody according to item [52], containing a heavy-chain variable region having an amino acid sequence set forth in SEQ ID NO: 99 and a light-chain variable region having an amino acid sequence set forth in SEQ ID NO: 103.
[55] A method for obtaining (selecting or identifying) an antibody that binds to an scFv, wherein
   the scFv contains a first region, a linker, and a second region and the first region, the linker, and the second region are arranged in this order in the direction from the N terminal to the C terminal of the scFv; and the first region and the second region are a heavy-chain variable region of an antibody and a light-chain variable region of an antibody, respectively, or a light-chain variable region of an antibody and a heavy-chain variable region of an antibody, respectively, and
   the method includes selecting or identifying an antibody that binds to the scFv from the group of antibodies, and the group of antibodies is the group of antibodies that compete with the antibody according to item [53] for binding to the scFv.
[56] A method for obtaining (or selecting or identifying) an antibody that binds to an scFv, wherein
   the scFv contains a first region, a linker, and a second region and the first region, the linker, and the second region are arranged in the scFv in this order in the direction from the N terminal to the C terminal; and
   the first region and the second region are a heavy-chain variable region of an antibody and a light-chain variable region of an antibody, respectively, or a light-chain variable region of an antibody and a heavy-chain variable region of an antibody, respectively; and
   the method includes selecting or identifying an antibody that binds to the scFv from the group of antibodies, and the group of antibodies is the group of antibodies that complete with the antibody according to item [54] for binding to the scFv.
[57] The antibody according to any one of the above items, wherein the antibody does not substantially bind to a naturally occurring (*in vivo*) immunoglobulin.
[58] The antibody according to any one of the above items, wherein the antibody does not substantially bind to a VH-L1-VL scFv and binds to a VL-L1-VH scFv.
[59] The antibody according to any one of the above items, wherein the antibody does not substantially bind to a VL-L1-VH scFv and binds to a VH-L1-VL scFv.
[60] The antibody according to any one of the above items, wherein the antibody does not substantially bind to a VH-L2-VL scFv and binds to a VL-L2-VH scFv.
[61] The antibody according to any one of the above items, wherein the antibody does not substantially bind to a VL-L2-VH scFv and binds to a VH-L2-VL scFv.
[62] The antibody according to item [58], wherein the antibody does not substantially bind a naturally occurring (*in vivo)* immunoglobulin.
[63] The antibody according to item [59], wherein the antibody does not substantially bind a naturally occurring (*in vivo)* immunoglobulin.
[64] The antibody according to item [60], wherein the antibody does not substantially bind a naturally occurring (*in vivo)* immunoglobulin.
[65] The antibody according to item [61], wherein the antibody does not substantially bind a naturally occurring (*in vivo)* immunoglobulin.
[66] The antibody according to any one of the above items, wherein the antibody is a human chimeric antibody, a humanized antibodies or a human antibody.
[67] The antibody according to any one of the above items, wherein the antibody is a non-human animal antibody.
[68] A composition containing the antibody according to any one of the above items.
[69] A composition containing the antibody according to item [57].
[70] The composition according to item [68], for use in detecting an scFv.
[71] The composition according to item [68], for use in detecting an scFv in a human body.
[72] The composition according to item [68], for use in targeting an scFv in a human body.
[73] The composition according to item [68], for use in detecting an scFv *in vivo.*
[74] The composition according to item [69], for use in detecting an scFv.
[75] The composition according to item [69], for use in detecting an scFv in a human body.
[76] The composition according to item [69], for use in targeting an scFv in a human body.
[77] The composition according to item [69], for use in detecting an scFv *in vivo.*
[78] The composition containing an antibody according to any one of items [62] to [65].
[79] The composition according to item [78], for use in detecting an scFv.
[80] The composition according to item [78], for use in detecting an scFv in a human body.
[81] The composition according to item [78], for use in targeting an scFv in a human body.
[82] The composition according to item [78], for use in detecting an scFv *in vivo.*

### Brief Description of Drawings

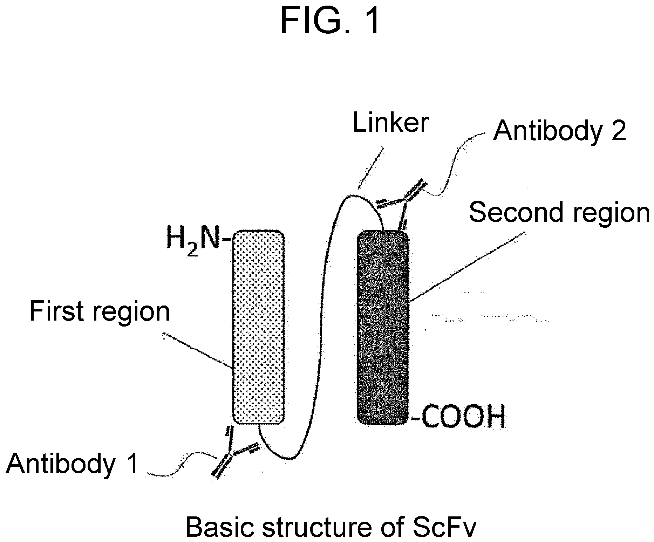

[Figure 1] Figure 1 shows a structure of a typical scFv and examples of the antibody of the present invention, antibodies 1 and 2. In Figure 1, antibody 1 binds to a linking part between a first region and a linker and antibody 2 binds to a linking part between the linker and a second region.

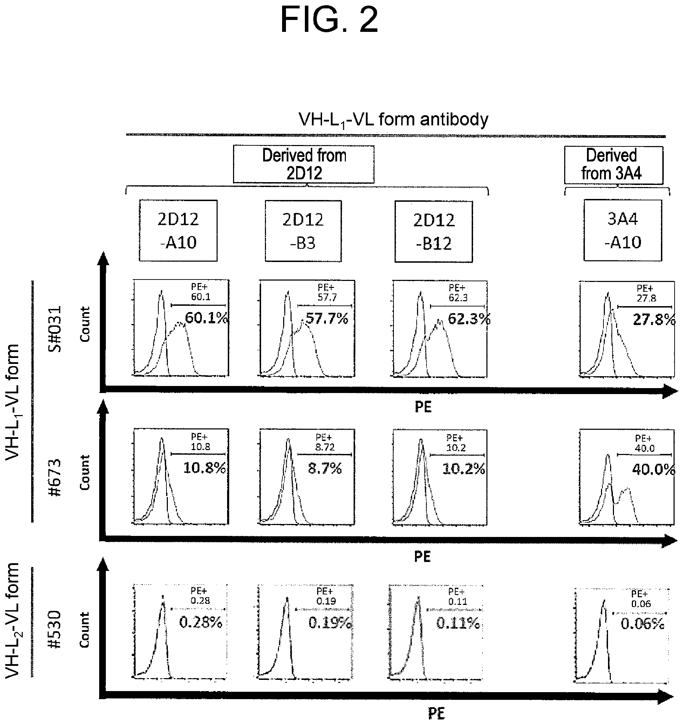

[Figure 2] Figure 2 shows the binding affinity of the exemplary antibodies according to the present invention for T cells expressing chimeric antigen receptors (CAR) containing different scFvs, which are examples of an antigen-binding compound containing an scFv. In Figure 2, antibodies against a VH-L₁-VL scFv were recovered from the culture supernatants of hybridoma clones and the binding ability of them to CAR-T cells were examined. In Figure 2, the stronger the binding affinity, the stronger the strength of PE. As the CAR-T cells, S#031, #673, and #530 were used. The details of these CAR-T cells are as shown in Table 5.

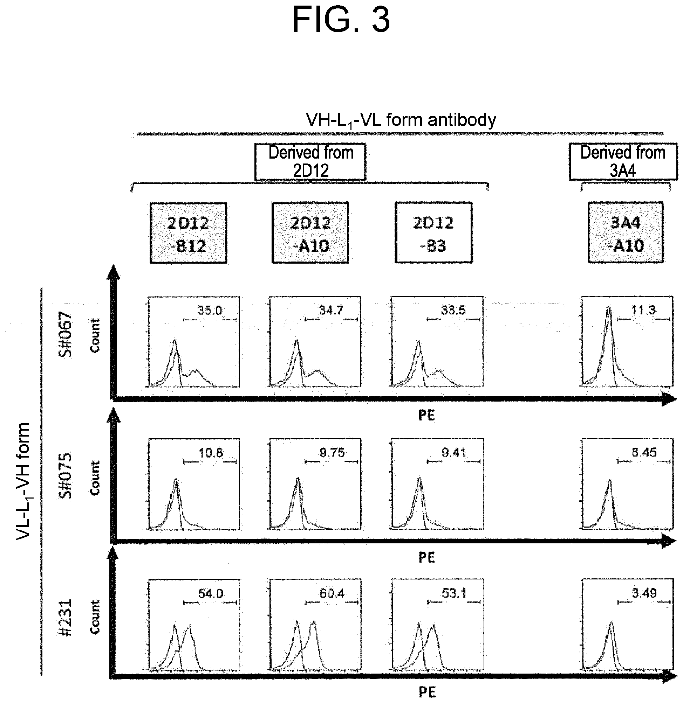

[Figure 3] Figure 3 shows the binding affinity of the exemplary antibodies according to the present invention for T cells expressing chimeric antigen receptors (CAR) containing different scFvs, which are examples of an antigen-binding compound containing an scFv. In Figure 3, antibodies against a VH-L₁-VL scFv were collected from the culture supernatants of hybridoma clones and the binding ability of them to CAR-T cells were examined. In Figure 3, the stronger the binding affinity, the stronger the strength of PE. As the CAR-T cells, S#067, S#075, and #231 were used. The details of these CAR-T cells are as shown in Table 5.

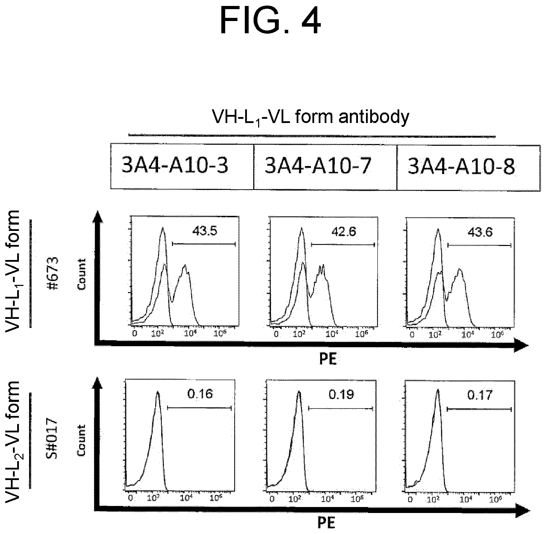

[Figure 4] Figure 4 shows the binding affinity of the exemplary antibodies according to the present invention for T cells expressing chimeric antigen receptors (CAR) containing different scFvs, which are examples of an antigen-binding compound containing an scFv. In Figure 4, antibodies against a VH-L₁-VL scFv were collected from the culture supernatants of hybridoma clones and the binding ability of them to CAR-T cells were examined. In Figure 4, the stronger the binding affinity, the stronger the strength of PE. As the CAR-T cells, #673, and S#017 were used. The details of these CAR-T cells are as shown in Table 5.

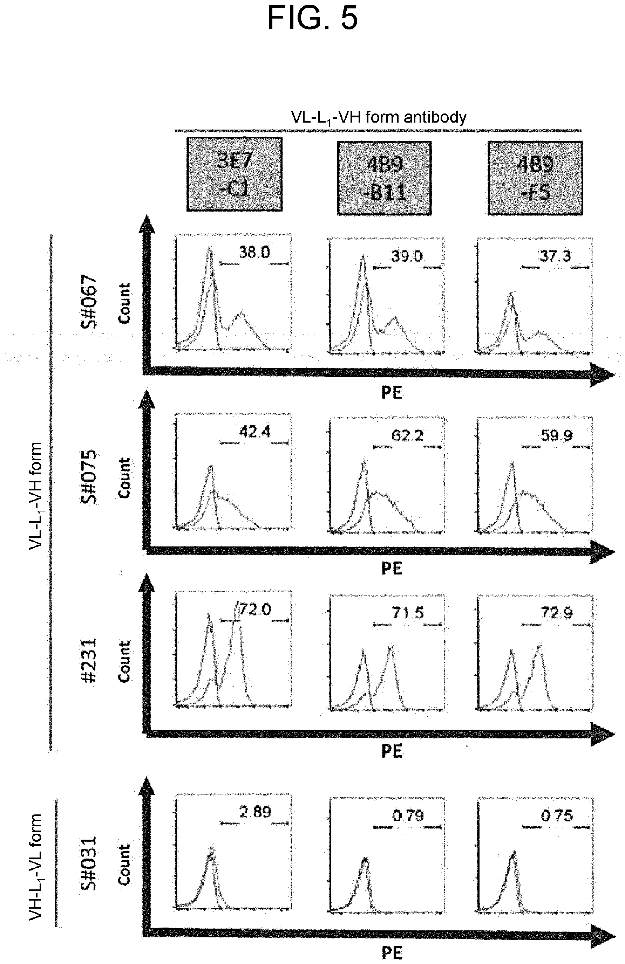

[Figure 5] Figure 5 shows the binding affinity of the antibodies exemplified by the present invention for T cells expressing chimeric antigen receptors (CAR) containing different scFvs, which are examples of an antigen-binding compound containing an scFv. In Figure 4, antibodies against a VL-L₁-VH scFv were collected from the culture supernatants of hybridoma clones and the binding ability of them to CAR-T cells were examined. In Figure 5, the stronger the binding affinity, the stronger the strength of PE. As the CAR-T cells, S#067, S#075, #231, and S#031 were used. The details of these CAR-T cells are as shown in Table 5.

### Detailed Description of the Invention

### <Definition>

In the specification, the term "comprise" is used in a sense covering the meaning expressed by "consist of". The term "comprise" may include elements other than the elements specified, whereas the term "consist of" does not essentially include elements other than the elements specified and the term "consists only of" does not include elements other than the elements specified.

In the specification, the term "subject" refers to a mammal. Examples of the mammal may include a rodent (for example, a mouse and a rat), a dog, a cat, a cow, a horse, a pig, a primate (for example, a monkey, a gorilla, an orangutan, a bonobo, a chimpanzee and a human). A human can be a typical example of the subject.

In the specification, the term "treatment" is used in a sense containing a therapy (therapeutic treatment) and a prophylaxis (preventive treatment). In the specification, the term "therapy" refers to remedy, curing or prevention of a disease or a disorder; improvement of a remission; or slow-down of progression of a disease or a disorder. In the specification, the term "prophylaxis" means decreasing the possibility of developing a disease or a pathological condition or delaying the onset of a disease or a pathological condition.

In the specification, the term "disease" refers to a symptom that benefits from a therapy. In the specification, the term "cancer" refers to a malignant tumor.

In the specification, the phrase "therapeutically effective amount" refers to the dose of a medicinal agent effective for treating (preventing or remedying) a disease or a condition. The therapeutically effective amount of a medicinal agent can slow down the progression of the symptom of a disease or a condition, stopping exacerbation of the symptom, improving the symptom, curing a symptom, or suppressing the onset or development of the symptom.

In the specification, the term "antibody" refers to an immunoglobulin. Antibodies generally have a structure composed of two heavy-chains (H-chains) and two light-chains (L-chains), which are associated and stabilized by pairs of disulfide bonds. The heavy-chain consists of a heavy-chain variable region VH, heavy-chain constant regions CH1, CH2 and CH3 and a hinge region positioned between CH1 and CH2. The light-chain consists of a light-chain variable region VL and a light-chain constant region CL. Of these, VH and VL form a variable region fragment (Fv), which is directly involved in antigen-binding and responsible for diversifying the antibody. An antigen-binding region consisting of VL, CL, VH and CH1 is called a Fab region. The region consisting of a hinge region, CH2 and CH3 is called an Fc region. In the variable regions, the regions directly in contact with an antigen are particularly variable and are called a complementarity-determining region (CDR). A portion that is a relatively less variable region except CDR is called a framework region (FR). Three CDRs are present in each of the heavy-chain and light-chain variable regions and individually called heavy-chain CDR1 to 3 and light-chain CDR1 to 3 in the order from the N terminal. An antibody may have various isotypes such as IgG. An antibody can be a human chimeric antibody, a humanized antibody, or a human antibody. A human chimeric antibody can be prepared by replacing a constant region of a non-human antibody with a constant region of a human antibody. A humanized antibody can be prepared by replacing the region except 6 CDRs of a non-human antibody with the corresponding regions of a human antibody. A human antibody can be prepared by using an animal (e.g., mouse) which has been prepared by replacing at least a heavy-chain variable region of an immunoglobulin with the corresponding region of a human locus. If the non-human derived constant region is used, the constant region is replaced with the amino acid sequence of a human antibody to obtain a human antibody. Generally, an antibody can selectively or specifically bind to its antigen.

In the specification, "CDR" refers to a complementarity-determining region present within a heavy-chain variable region and a light-chain variable region of an antibody. Three CDRs are present in each of a heavy-chain variable region and a light-chain variable region and called CDR1, CDR2, and CDR3 sequentially from the N terminal. The CDRs present in a heavy-chain variable region are called heavy-chain CDR1 to 3 (HCDR1 to 3). The CDRs present in a light-chain variable region are called light-chain CDR1 to 3 (LCDR1 to 3). The CDRs can be determined based on, for example, the Kabat numbering scheme (Kabat, E. A. et al., Sequences of Proteins of Immunological Interest, 5th ed., 1991, Bethesda: US Dept. of Health and Human Services, PHS, NIH.), Chothia, AbM, contact, IMGT, Aho, or Martin (Enhanced Chothia). As mentioned above, heavy-chain CDR1 to 3 and light-chain CDR1 to 3 can be estimated based on the amino acid sequences of a heavy-chain variable region and a light-chain variable region in accordance with a routine method.

In the specification, "scFv" is an antigen-binding fragment of an antibody having a heavy-chain variable region or an antigen-binding region thereof and a light-chain variable region or an antigen-binding region thereof. In the scFv, the heavy-chain variable region or an antigen-binding region thereof and the light-chain variable region or an antigen-binding region thereof are linked via a linker. In the specification, the scFv has a first region, a linker, and a second region in this order in the direction from the N terminal to the C terminal, as shown in Figure 1. The first region can be a heavy-chain variable region or an antigen-binding region thereof, and the second region can be a light-chain variable region or an antigen-binding region thereof. Alternatively, the first region can be a light-chain variable region or an antigen-binding region thereof, and the second region can be a heavy-chain variable region or an antigen-binding region thereof. As the linker, generally, a flexible linker is preferably used. In the scFv, the heavy-chain variable region or an antigen-binding region thereof and the light-chain variable region or an antigen-binding region thereof may contain a sequence derived from an antibody of an animal immunized. In the scFv, the heavy-chain variable region or an antigen-binding region thereof and the light-chain variable region or an antigen-binding region thereof may be humanized. In the scFv, the heavy-chain variable region or an antigen-binding region thereof and light-chain variable region or an antigen-binding region thereof can be a heavy-chain variable region or an antigen-binding region thereof and a light-chain variable region or an antigen-binding region thereof of a human antibody. An scFv is sometimes incorporated in an antigen-binding compound as a part thereof and serves as an antigen-binding site therein.

In the specification, examples of the "antibody that binds to the linking part between a first region and a linker" may include an antibody binding only to the first region, an antibody binding only to the linker, and an antibody that binds to both the first region and the linker (for example, an antibody that binds to neither a construct formed only of the linker nor a construct formed only of the first region). In the specification, examples of the "antibody that binds to the linking part between a second region and a linker" may include an antibody that binds only to the second region, an antibody binding only to the linker, and an antibody that binds to both the second region and the linker (for example, an antibody that binds to neither a construct formed only of the linker nor a construct formed only of the second region). In the specification, the "linking part" used in the "linking part between a first region and a linker", the "linking part between a second region and a linker" and similar phrases, contains a framework region of an antigen-binding fragment (for example, scFv) of an antibody and a linker. An antibody that binds to a construct formed only of a framework may possibly bind to a naturally occurring immunoglobulin. For example, when an antibody to be administered *in vivo* is required not to substantially bind to a naturally occurring immunoglobulin, it is preferred that the antibody does not substantially bind to the naturally occurring immunoglobulin, and, for example, the antibody does not substantially bind to a construct formed only of a framework region. The antibody to be administered to an individual is preferably an antibody that binds, particularly, to a linking part between a second region and a linker but not to a construct formed only of a linker, a region formed only of a first region or a construct formed only of a second region. The binding property of an antibody can be confirmed by a test for examining the binding between peptides containing individual regions and the antibody. The antibody to be administered to an individual is preferably formed by replacing the sequence except for CDRs of a fragment of the antibody with the sequence of the individual (to which the antibody is to be administered). In this regard, the antibody to be administered to a human can be preferably a human chimeric antibody, more preferably a humanized antibody, and further preferably a human antibody. The antibodies to be used *in vitro* are not particularly restricted in such a way, and therefore, an antibody that binds to a linking part (for example, a non-human animal antibody, a non-human mammalian antibody, a human chimeric antibody, a humanized antibody, and a human antibody) can be preferably used *in vivo.*

In the specification, the term "isolation" means that a component is separated from the at least one or more of other components present in the same system.

In the specification, "chimeric antigen receptor" (CAR) is a chimeric molecule having an antigen-binding fragment (particularly, scFv) of an antibody and an immune-cell activation domain. The CAR is generally a molecule having an scFv, an extracellular hinge domain, a transmembrane domain (for example, CD8α or CD28), and an activation signaling domain (for example, CD3ζ) linked. A CAR can be introduced into a cell and expressed on the cell surface. The cell having a CAR expressed thereon can be targeted to a predetermined antigen. When a CAR is introduced, for example, in an immune cell such as a T cell or an NK cell, the immune cell (such as a T cell or an NK cell) can be targeted to cancer. The first-generation CAR had an scFv, an extracellular hinge domain, a transmembrane domain (for example, CD8α or CD28), and an activation signaling domain (for example, CD3ζ) linked therein, whereas the second-generation CAR further contains a costimulatory molecule signaling domain for activating an immune cell having CAR introduced therein. Examples of the costimulatory molecule signaling domain include costimulatory factors such as CD28, 4-1BB, OX40, CD27, and ICOS. In the third-generation CAR, a plurality of costimulatory factors are integrated therein. As mentioned above, a CAR has been improved in order to continuously proliferate an immune cell having the CAR introduced therein within a living organism. All domains other than an scFv portion are preferably derived from human protein.

### <Antibody of the present invention capable of detecting scFv>

According to the present invention, there is provided an antibody that binds to an scFv. The antibody of the present invention can be designed so as to bind to a portion near linker (particularly, a linking part between a first region and a linker, or a linking part between a linker and a second region) in order to bind to scFvs to various antigens.

In all embodiments, an scFv may preferably have framework regions of a human heavy-chain variable region and a human light-chain variable region. In a preferable embodiment, an scFv may have a human-antibody-derived heavy-chain variable region and light-chain variable region.

### <1> Antibody that binds to the linking part between the first region and linker

The present inventors have found that an antibody that can be obtained by using, as an antigen, an isolated short-chain peptide (for example, a 10 to 20 amino acid length, more specifically, 13 to 20 amino acids), which has an amino acid sequence of a region that is in the first region and has 1 to 10 amino acids from the C terminal of the first region, can bind to scFvs to various antigens. When a flexible linker is used as the linker, it is not always easy to obtain an antibody that binds to the linker. However, according to the present invention, even in the case where a flexible linker is used as the linker, it is possible to satisfactorily obtain an antibody that binds to a portion near the linker due to the immunogenicity of the first region or second region.

In other words, the present invention provides an antibody that binds to an isolated short-chain peptide containing a region present within a first region and having 1 to 10 amino acids from the C terminal of the first region. The short-chain peptide can be preferably a partial peptide of an scFv.

The amino acid sequence of a heavy-chain variable region near the C terminal from a heavy-chain CDR3 (HCDR3) is selected from the group consisting of, for example, WGQGTLVTVSS (SEQ ID NO: 1), WGQGTLVTVSS (SEQ ID NO: 2), WGQGTLVTVSS (SEQ ID NO: 3), WGQGTTLTVS (SEQ ID NO: 4), WGQGTTVTVSSGILGS (SEQ ID NO: 5), GQGTLVTVSS (SEQ ID NO: 109), GAGTTVTVSS (SEQ ID NO: 110), GQGTLVTVSA (SEQ ID NO: 111), GQGTLVTVSS (SEQ ID NO: 117), GQGTTVTVSSGILGS (SEQ ID NO: 118), and GQGTLVTVSS (SEQ ID NO: 119). Accordingly, the short-chain peptide can be designed so as to contain 1 to 10 amino acids from the C terminal of one of these sequences preferably 1 to 5 amino acids, for example, 3 to 5 amino acids (for example, VSS, TVSS, VTVSS (SEQ ID NO: 6), or VTVSA (SEQ ID NO: 120); TVS, LTVS, or TLTVS (SEQ ID NO: 79); or LGS, ILGS, or GILGS (SEQ ID NO: 80)). The antibody can bind to a region containing 1 to 10 amino acids from the C terminal of the first region, and preferably, 1 to 5 amino acids (for example, a single amino acid, 1 to 2 amino acids, 1 to 3 amino acids, 1 to 4 amino acids, or 1 to 5 amino acids) from the C terminal of the first region.

The amino acid sequence of a light-chain variable region near the C terminal from a light-chain CDR3 (HCDR3) can be selected from the group consisting of, for example, FGGGTKLEIK (SEQ ID NO: 7), FGAGTKLELKR (SEQ ID NO: 8), FGGGTQLTVLS (SEQ ID NO: 9), FGGGTKVEIK (SEQ ID NO: 81), FGGGTKVEIK (SEQ ID NO: 107), FGGGTKLEIK (SEQ ID NO: 108), FGGGTKVEIK (SEQ ID NO: 112), FGGGTKLEIK (SEQ ID NO: 113), FGGGTKLEIK (SEQ ID NO: 114), FGGGTKLEIK (SEQ ID NO: 115), and FGGGTKVEIK (SEQ ID NO: 116). Accordingly, the short-chain peptide can be designed so as to contain 1 to 10 amino acids from the C terminal of one of these sequences, preferably 1 to 5 amino acids, for example, 3 to 5 amino acids (for example, EIK, LEIK, or KLEIK (SEQ ID NO: 10); LKR, ELKR, or LELKR (SEQ ID NO: 11); VLS, TVLS, or LTVLS (SEQ ID NO: 12); or EIK, VEIK, or KVEIK (SEQ ID NO: 82)). The antibody can bind to a region containing 1 to 10 amino acids from the C terminal of the first region, and preferably, 1 to 5 amino acids (for example, a single amino acid, 1 to 2 amino acids, 1 to 3 amino acids, 1 to 4 amino acids, or 1 to 5 amino acids) from the C terminal of the first region.

In a preferable embodiment of the present invention, an antibody can be obtained by using, as an immunogen, a peptide having an amino acid sequence present in (a) a region of 1 to 10 amino acids from the C terminal of the first region and (b) a partial region of a linker continuous with the region and having 1 to 10 amino acids from the N terminal of the linker. In this manner, an antibody against a linking part between a first region and the linker can be obtained. In the embodiment, the isolated short-chain peptide can be designed so as to contain (a) a region of 1 to 10 amino acids from the C terminal of the first region, preferably 1 to 5 amino acids, for example, 3 to 5 amino acids (for example, VTV, TVS, VSS, VTVS, TVSS, VTVSS (SEQ ID NO: 6), or VTVSA (SEQ ID NO: 120); TVS, LTVS, or TLTVS (SEQ ID NO: 79); or LGS, ILGS, or GILGS (SEQ ID NO: 80)). In the embodiment, the isolated short-chain peptide can be designed so as to contain (b) a partial region of a linker continuous with the region and having 1 to 10 amino acids from the N terminal of the linker, preferably 1 to 8 amino acids, for example, 3 to 8 amino acids (for example, a single amino acid, 2 amino acids, 3 amino acids, 4 amino acids, 5 amino acids, 6 amino acids, 7 amino acids, or 8 amino acids). In the embodiment, the isolated short-chain peptide can be designed so as to contain (b) a region of 1 to 10 amino acids from the N terminal of the linker, preferably 1 to 8 amino acids, for example, 3 to 8 amino acids (for example, 3 amino acids, 4 amino acids, 5 amino acids, 6 amino acids, 7 amino acids, or 8 amino acids such as GGG, GGGG, GGGGS (SEQ ID NO: 13) or GGGGSG (SEQ ID NO: 14); or SSA, SAD, SSAD, SADD, SSADD (SEQ ID NO: 15), SSADDA (SEQ ID NO: 16), SSADDAK (SEQ ID NO: 17), or SSADDAKK (SEQ ID NO: 18)). In an embodiment, the isolated short-chain peptide may contain 1 to 5 amino acids from the C terminal of the first region and 3 to 8 amino acids from the N terminal of the linker. In a preferable embodiment, the isolated short-chain peptide may be a peptide containing at least 3 to 5 amino acids (for example, VTV, TVS, VSS, VTVS, TVSS, or VTVSS (SEQ ID NO: 6)) from the C terminal of the first region a and at least 3 to 8 amino acids from the N terminal side of a linker (for example, GGG, GGGG, GGGGS (SEQ ID NO: 13) or GGGGSG (SEQ ID NO: 14), SSA, SAD, SSAD, SADD, or SSADD (SEQ ID NO: 15), SSADDA (SEQ ID NO: 16), SSADDAK (SEQ ID NO: 17), or SSADDAKK (SEQ ID NO: 18)).

In a preferable embodiment, the isolated short-chain peptide may contain any one of Nos. 1 to 16 in Table 1 below.

**[Table 1]**

| Table 1: Preferable region the short-chain peptide may at least have | | | | | |
|---|---|---|---|---|---|
| No. | From C terminal of 1st region | From N terminal of linker | No. | From C terminal of 1st region | From N terminal of linker |
| 1 | 8 amino acids | 8 amino acids | 15 | 3 amino acids | 6 amino acids |
| 2 | 7 amino acids | 8 amino acids | 16 | 6 amino acids | 5 amino acids |
| 3 | 6 amino acids | 8 amino acids | 17 | 6 amino acids | 4 amino acids |
| 4 | 5 amino acids | 8 amino acids | 18 | 6 amino acids | 3 amino acids |
| 5 | 4 amino acids | 8 amino acids | 19 | 5 amino acids | 5 amino acids |
| 6 | 3 amino acids | 8 amino acids | 20 | 4 amino acids | 5 amino acids |
| 7 | 7 amino acids | 7 amino acids | 21 | 3 amino acids | 5 amino acids |
| 8 | 6 amino acids | 7 amino acids | 22 | 5 amino acids | 4 amino acids |
| 9 | 5 amino acids | 7 amino acids | 23 | 5 amino acids | 3 amino acids |
| 10 | 4 amino acids | 7 amino acids | 24 | 4 amino acids | 4 amino acids |
| 11 | 3 amino acids | 7 amino acids | 25 | 3 amino acids | 4 amino acids |
| 12 | 6 amino acids | 6 amino acids | 26 | 4 amino acids | 3 amino acids |
| 13 | 5 amino acids | 6 amino acids | 27 | 3 amino acids | 3 amino acids |
| 14 | 4 amino acids | 6 amino acids | | | |

In a preferable embodiment, the isolated short-chain peptide may contain the amino acid sequence of SEQ ID NO: 19: VTVSSSSADDAKK. In the embodiment, the short-chain peptide may contain a region having the C terminal of a heavy-chain variable region and the N terminal of a linker linked therein. In a preferable embodiment, each of the N terminal and C terminal of the isolated short-chain peptide disclosed in the specification, 1 to 3 amino acids (for example, two amino acids such as GG and a single amino acid such as C) may be added.

In a preferable embodiment, the isolated short-chain peptide may contain the amino acid sequence of SEQ ID NO: 20: VTVSSGGGGSG.
In the embodiment, the short-chain peptide may contain a region having the C terminal of a heavy-chain variable region and the N terminal of a linker linked therein.

In a preferable embodiment, the isolated short-chain peptide can be the amino acid sequence of SEQ ID NO: 21: GGVTVSSSSADDAKKC or the amino acid sequence of SEQ ID NO: 22: GGVTVSSGGGGSGC.

In a preferable embodiment, the isolated short-chain peptide may contain the amino acid sequence of SEQ ID NO: 23: LEIKSSADDAKK. In the embodiment, the short-chain peptide may contain a region having the C terminal of a light-chain variable region and the N terminal of a linker linked therein.

In a preferable embodiment, the isolated short-chain peptide contain the amino acid sequence of SEQ ID NO: 24: LEIKGGGGSG. In the embodiment, the short-chain peptide may contain a region having the C terminal of a light-chain variable region and the N terminal of a linker linked therein.

In a preferable embodiment, the isolated short-chain peptide can be the amino acid sequence of SEQ ID NO: 70: GGLEIKSSADDAKKC, or the amino acid sequence of SEQ ID NO: 71: GGLEIKGGGGSGC.

In a preferable embodiment, the antibody of the present invention can bind to an isolated short-chain peptide as mentioned above and can bind to an scFv. In an embodiment, an scFv can be present in the form of being incorporated into another antigen-binding compound (for example, CAR molecule). As the antigen-binding compound, all protein molecules containing an scFv as an antigen-binding portion may be exemplified. Examples of the antigen-binding compound include, but are not particularly limited to, a CAR, an IgG-scFv (a molecule in which an scFv is linked to the Fc region of an IgG molecule), an scFv-Fc (a molecule in which a variable region of an antibody is replaced with an scFv), an scFv₂-Fc (a molecule in which a variable region of an antibody is replaced with an scFv and an scFv is linked to the Fc region thereof), an scFv-HSA-scFv (a molecule in which an scFv is linked to each of the N terminal and C terminal of human serum albumin), a Fab-scFv-Fc (a molecule in which variable regions of an antibody are replaced with a Fab and an scFv, respectively, and a Fab and an scFv are linked to the Fc region), a Fab-scFv (a molecule in which an scFv is linked to a Fab), and a BiTE (molecules in which two scFvs are linked).

In an embodiment, a linker may have a 10 to 40 amino acid length, for example, 15 to 25 amino acid length.

In an embodiment, the linker in an scFv can be a flexible linker. Examples of the flexible linker include a linker containing G and S, such as GGGGSGGGGS (SEQ ID NO: 25), GGGGSGGGGSGGGGS (SEQ ID NO: 26), GGGGSGGGGSGGGGSGGGGS (SEQ ID NO: 27) and GGGGSGGGGSGGGGSGGGGSGGGGS (SEQ ID NO: 28); and SSADDAKKDAAKKDDAKKDDAKKDG (SEQ ID NO: 29).

In an embodiment, the antibody of the present invention can bind to an isolated short-chain peptide as mentioned above and can bind to an scFv. In addition, the antibody of the present invention can recognize a linking part between a first region and a linker in the isolated short-chain peptide.

In an embodiment, the antibody of the present invention binds to a peptide containing only a first region, and a peptide containing only a linker with a lower binding affinity than that for an isolated short-chain peptide as mentioned above (for example, 50% or less, 40% or less, 30% or less, 20% or less, 10% or less, or 1% or less, 1/1000 or less, or 1/10000 or less) or does not have a significant binding affinity. Alternatively, the antibody of the present invention binds to an scFv in which the order of the heavy-chain variable region and the light-chain variable region (i.e., the first region and the second region) is interchanged with a lower binding affinity than that for an scFv in which the first region, linker and second region are linked in this order (for example, 50% or less, 40% or less, 30% or less, 20% or less, 10% or less, or 1% or less, 1/1000 or less, or 1/10000 or less) or does not have a significant binding affinity. Alternatively, the lower binding affinity may mean that binding dissociation constant KD is more than 1 (exceeding the KD value of a comparative subject), 1.1 times or more, 1.2 times or more, 1.3 times or more, 1.4 times or more, 1.5 times or more, 2 times or more, 3 times or more, 4 times or more, 5 times or more, 6 times or more, 7 times or more, 8 times or more, 9 times or more, 10 times or more, 100 times or more, or 1000 times or more as large as the KD value of a comparative subject.

### <2> Antibody that binds to linking part between linker and second region

In the above section, an antibody that binds to the linking part between a first region and a linker was described. However, an antibody that binds to the linking part between a linker and a second region can also be useful like the above.

In an embodiment of the present invention, there can be provided an antibody that binds to an isolated short-chain peptide that is present in the second region and contains 1 to 10 amino acids from the N terminal of the second region.

In an embodiment of the present invention, there can be provided an antibody that binds to an isolated short-chain peptide (for example, a 10 to 20 amino acid length, more specifically 13 to 20 amino acids) having amino acid sequences of (c) a partial region of a linker continuous with a second region and having 1 to 10 amino acids from the C terminal of the linker, and (d) a region present within the second region and having 1 to 10 amino acids from the N terminal of the second region.

Examples of the amino acid sequence of a heavy-chain variable region closer to the N terminal than heavy-chain CDR1 (HCDR1) may include QVTLKESGPA (SEQ ID NO: 30), QVQLVQSGAE (SEQ ID NO: 31), EVQLVQSGAE (SEQ ID NO: 32), SQVQLKESGP (SEQ ID NO: 33), and QVQLQQSGPG (SEQ ID NO: 34). Accordingly, the short-chain peptide can be designed so as to contain 1 to 10 amino acids, preferably 1 to 5 amino acids, for example, 3 to 5 amino acids (for example, VTVSS (SEQ ID NO: 6)) from the C terminal of one of these sequences. The antibody can bind to a region containing 1 to 10 amino acids from the C terminal of the first region, and preferably, 1 to 5 amino acids (for example, 1 amino acid, 1 to 2 amino acids, 1 to 3 amino acids, 1 to 4 amino acids, or 1 to 5 amino acids) from the C terminal of the first region.

Examples of the amino acid sequence of a heavy-chain variable region closer to the N terminal than light-chain CDR1 include DIVMTQSPLS (SEQ ID NO: 35), DIVMTQSPDS (SEQ ID NO: 36), DIQLTQSPSS (SEQ ID NO: 37), DIQMTQSPKF (SEQ ID NO: 38), and QPVLTQSSSL (SEQ ID NO: 39). Accordingly, the short-chain peptide can be designed so as to contain 1 to 10 amino acids, preferably 1 to 5 amino acids, for example, 3 to 5 amino acids (for example, VTVSS (SEQ ID NO: 6)) from the N terminal of each of these sequences. The antibody can bind to a region containing 1 to 10 amino acids from the N terminal of the second region, and preferably, 1 to 5 amino acids (for example, 1 amino acid, 1 to 2 amino acids, 1 to 3 amino acids, 1 to 4 amino acids, or 1 to 5 amino acids) from the N terminal of the second region.

In a preferable embodiment of the present invention, there can be provided an antibody using, as an immunogen, a peptide having amino acid sequences of (c) a partial region of a linker continuous with a second region and having 1 to 10 amino acids from the C terminal of the linker, and (d) a region present within the second region and having 1 to 10 amino acids from the N terminal of the second region. In this way, an antibody against a linking part between a second region and a linker can be obtained. In the embodiment, the isolated short-chain peptide can be designed so as to contain (c) a partial region of a linker continuous with a second region and having 1 to 10 amino acids from the C terminal of the linker, preferably 1 to 8 amino acids, more specifically, 3 to 8 amino acids (3 amino acids, 4 amino acids, 5 amino acids, 6 amino acids, 7 amino acids or 8 amino acids such as GGS, GGGS, GGGGS (SEQ ID NO: 13), or SGGGGS (SEQ ID NO: 40), or KDG, KKDG, AKKDG (SEQ ID NO: 41), DAKKDG (SEQ ID NO: 42), DDAKKDG (SEQ ID NO: 43), or KDDAKKDG (SEQ ID NO: 44)). In the embodiment, the isolated short-chain peptide can be designed so as to contain (d) a region present within the second region and having 1 to 10 amino acids, preferably 1 to 5 amino acids, for example, 3 to 5 amino acids (for example, DX₁X₂X₃T, in which X₁ is I or P, X₂ is V or Q, X₃ is M or L (SEQ ID NO: 45), for example, DIVMT (SEQ ID NO: 46), DIQLT (SEQ ID NO: 47), DIQMT (SEQ ID NO: 48), or QPVLT (SEQ ID NO: 49)) from the N terminal of the second region. In this embodiment, the isolated short-chain peptide may contain 3 to 8 amino acids from the C terminal of the linker and 1 to 5 amino acids from the N terminal of the second region. In a preferable embodiment, examples of the isolated short-chain peptide include a peptide containing 3 to 8 amino acids (for example, GGS, GGGS, GGGGS (SEQ ID NO: 13), or SGGGGS (SEQ ID NO: 40), or KDG, KKDG, AKKDG (SEQ ID NO: 41), DAKKDG (SEQ ID NO: 42), DDAKKDG (SEQ ID NO: 43), or KDDAKKDG (SEQ ID NO: 44)) from the C terminal of the linker and a peptide containing 3 to 5 amino acids from the N terminal of a second region.

In a preferable embodiment, the isolated short-chain peptide may contain any one of Nos. 1 to 27 in Table 2 below.

**[Table 2]**

| Table 2: Preferable region the short-chain peptide may at least have | | | | | |
|---|---|---|---|---|---|
| No. | From C terminal of linker | From N terminal of second region | No. | From C terminal of linker | From N terminal of second region |
| 1 | 8 amino acids | 8 amino acids | 15 | 3 amino acids | 6 amino acids |
| 2 | 7 amino acids | 8 amino acids | 16 | 6 amino acids | 5 amino acids |
| 3 | 6 amino acids | 8 amino acids | 17 | 6 amino acids | 4 amino acids |
| 4 | 5 amino acids | 8 amino acids | 18 | 6 amino acids | 3 amino acids |
| 5 | 4 amino acids | 8 amino acids | 19 | 5 amino acids | 5 amino acids |
| 6 | 3 amino acids | 8 amino acids | 20 | 4 amino acids | 5 amino acids |
| 7 | 7 amino acids | 7 amino acids | 21 | 3 amino acids | 5 amino acids |
| 8 | 6 amino acids | 7 amino acids | 22 | 5 amino acids | 4 amino acids |
| 9 | 5 amino acids | 7 amino acids | 23 | 5 amino acids | 3 amino acids |
| 10 | 4 amino acids | 7 amino acids | 24 | 4 amino acids | 4 amino acids |
| 11 | 3 amino acids | 7 amino acids | 25 | 3 amino acids | 4 amino acids |
| 12 | 6 amino acids | 6 amino acids | 26 | 4 amino acids | 3 amino acids |
| 13 | 5 amino acids | 6 amino acids | 27 | 3 amino acids | 3 amino acids |
| 14 | 4 amino acids | 6 amino acids | | | |

In a preferable embodiment, the isolated short-chain peptide may contain
the amino acid sequence of SEQ ID NO: 50:
   KDDAKKDGQVTLK,
the amino acid sequence of SEQ ID NO: 51:
   KDDAKKDGQVQLV,
the amino acid sequence of SEQ ID NO: 52:
   KDDAKKDGEVQLV,
the amino acid sequence of SEQ ID NO: 53:
   KDDAKKDGSQVQL, or
the amino acid sequence of SEQ ID NO: 54:
   KDDAKKDGQVQLQ.

In the embodiment, the short-chain peptide contains a region having the C terminal of the linker and the N terminal of a heavy-chain variable region linked therein.

In a preferable embodiment, the isolated short-chain peptide may contain,
the amino acid sequence of SEQ ID NO: 55:
   SGGGGSQVTLK
the amino acid sequence of SEQ ID NO: 56:
   SGGGGSQVQLV,
the amino acid sequence of SEQ ID NO: 57:
   SGGGGSEVQLV,
the amino acid sequence of SEQ ID NO: 58:
   SGGGGSSQVQL, or
the amino acid sequence of SEQ ID NO: 59:
   SGGGGSQVQLQ.

In the embodiment, the short-chain peptide contains a region having the C terminal of the linker and the N terminal of a light-chain variable region linked therein.

In a preferable embodiment, the isolated short-chain peptide may contain,
the amino acid sequence of SEQ ID NO: 83:
   KDDAKKDGDIVMT,
the amino acid sequence of SEQ ID NO: 84:
   KDDAKKDGDIQLT,
the amino acid sequence of SEQ ID NO: 85:
   KDDAKKDGDIQMT, or
the amino acid sequence of SEQ ID NO: 86:
   KDDAKKDGQPVLT.

In the embodiment, the short-chain peptide contains a region having the C terminal of the linker and the N terminal of a light-chain variable region linked therein.

In a preferable embodiment, the isolated short-chain peptide may contain,
the amino acid sequence of SEQ ID NO: 87:
   SGGGGSDIVMT,
the amino acid sequence of SEQ ID NO: 88:
   SGGGGSDIQLT,
the amino acid sequence of SEQ ID NO: 89:
   SGGGGSDIQMT, or
the amino acid sequence of SEQ ID NO: 90:
   SGGGGSQPVLT.

In the embodiment, the short-chain peptide contains a region having the C terminal of the linker and the N terminal of a light-chain variable region linked therein.

In a preferable embodiment, the isolated short-chain peptide can be,
the amino acid sequence of SEQ ID NO: 60:
   GGKDDAKKDGQVTLKC,
the amino acid sequence of SEQ ID NO: 61:
   GGKDDAKKDGQVQLVC,
the amino acid sequence of SEQ ID NO: 62:
   GGKDDAKKDGEVQLVC,
the amino acid sequence of SEQ ID NO: 63:
   GGKDDAKKDGSQVQLC, or
the amino acid sequence of SEQ ID NO: 64:
   GGKDDAKKDGQVQLQC.

In a preferable embodiment, the isolated short-chain peptide can be,
the amino acid sequence of SEQ ID NO: 65:
   GGSGGGGSQVTLKC,
the amino acid sequence of SEQ ID NO: 66:
   GGSGGGGSQVQLVC,
the amino acid sequence of SEQ ID NO: 67:
   GGSGGGGSEVQLVC,
the amino acid sequence of SEQ ID NO: 68:

   GGSGGGGSSQVQLC, or
the amino acid sequence of SEQ ID NO: 69:
   GGSGGGGSQVQLQC.

As described above, an antibody that binds to a linking part of a linker and a heavy-chain or light-chain variable region can be obtained by immunizing an animal with a peptide having the amino acid sequence of the linking part, and obtaining the antibody. In this case, even if a linker itself has low immunogenicity, an antibody that binds to a portion near the linker can be obtained.

In an embodiment, the antibody of the present invention binds to an isolated short-chain peptide as mentioned above but it binds to a peptide having only a second region and a peptide having only the sequence of a linker, only with a lower affinity than the isolated short-chain peptide (for example, 50% or less, 40% or less, 30% or less, 20% or less, 10% or less, or 1% or less, 1/1000 or less, or 1/10000 or less) or does not significantly bind to the peptide. The lower binding affinity may mean that binding dissociation constant KD is more than 1 (exceeding the KD value of a comparative subject), 1.1 times or more, 1.2 times or more, 1.3 times or more, 1.4 times or more, 1.5 times or more, 2 times or more, 3 times or more, 4 times or more, 5 times or more, 6 times or more, 7 times or more, 8 times or more, 9 times or more, 10 times or more, 100 times or more, or 1000 times or more as large as the KD value of a comparative subject.

In an embodiment, the antibody of the present invention binds to an isolated short-chain peptide as mentioned above but it binds to either one of a peptide only having a second region and a peptide only having the sequence of a linker, with a significant binding affinity.

### <3> Antibody binding principally to linker

In an embodiment, the antibody of the present invention can bind to an isolated short-chain peptide as mentioned above and can bind to an scFv. In an embodiment, the antibody of the present invention binds principally to either one of a first region and a linker. In an embodiment, the antibody of the present invention may an antibody that binds to an isolated short-chain peptide as mentioned above, and principally binds to the linker of an scFv. The antibody that principally binds to the linker of an scFv refers to an antibody binding even to an scFv in which the first and second regions are interchanged. In actual binding, the antibody needs not to bind only to a linker and may also bind to an amino acid in either one of the first and second regions that are positioned near the linker of the first and second region, respectively. In an embodiment, an antibody binding principally to the linker of an scFv can bind to a peptide consisting of the amino acid sequence of the linker.

In an embodiment, the antibody of the present invention can bind to any one of the peptides having amino acid sequences of SEQ ID NO: 13 to 18 and 26 to 29 and can bind to an scFv. In an embodiment, the antibody of the present invention can bind to a peptide having the amino acid sequence of SEQ ID NO: 13 or 14 and can bind to an scFv. In an embodiment, the antibody of the present invention can bind to any one of the peptides having the amino acid sequences of SEQ ID NO: 15 to 18, and can bind to an scFv. In an embodiment, the antibody of the present invention can bind to any one of the peptides having the amino acid sequences of SEQ ID NO: 26 to 28, and can bind to an scFv. In an embodiment, the antibody of the present invention can bind to a peptide having the amino acid sequence of SEQ ID NO: 29 and can bind to a scFv.

As shown in Examples, an animal is immunized with a peptide having the amino acid sequence of a linking part of a linker and a heavy-chain or light-chain variable region to obtain an antibody. As a result, even if a linker itself has low immunogenicity, an antibody binding principally to the linker can be obtained.

An antibody binding principally to a linker binds even to an scFv in which the first and second regions are interchanged. Since an antibody binding principally to a linker binds even to scFvs having the first and second regions different in sequence, the antibody finds a wide range of use.

### <4> Antibodies that binds to more specific peptides

In a preferable embodiment, the isolated short-chain peptide can be a peptide having the amino acid sequence set forth in SEQ ID NO: 19. In a preferable embodiment, the isolated short-chain peptide can be a peptide having the amino acid sequence set forth in SEQ ID NO: 20. In a preferable embodiment, the isolated short-chain peptide can be a peptide having the amino acid sequence set forth in SEQ ID NO: 21. In a preferable embodiment, the isolated short-chain peptide can be a peptide having the amino acid sequence set forth in SEQ ID NO: 22. In a preferable embodiment, the isolated short-chain peptide can be a peptide having the amino acid sequence set forth in SEQ ID NO: 23. In a preferable embodiment, the isolated short-chain peptide can be a peptide having the amino acid sequence set forth in SEQ ID NO: 24. In a more preferable embodiment, the antibody of the present invention can bind to one or more of the isolated short-chain peptides and to an antibody fragment containing an scFv as a partial structure. Examples of an antibody fragment containing an scFv as a partial structure includes any of protein molecules containing an scFv as mentioned above as an antigen-binding portion. Specifically, examples of the antibody fragment containing an scFv as a partial structure include, but are not particularly limited to, a CAR, an IgG-scFv (a molecule in which an scFv is linked to the Fc region of an IgG molecule), an scFv-Fc (a molecule in which a variable region of an antibody is replaced with an scFv), an scFv₂-Fc (a molecule in which a variable region of an antibody is replaced with an scFv and an scFv is linked to the Fc region thereof), an scFv-HSA-scFv (a molecule in which an scFv is linked to each of the N terminal and C terminal of human serum albumin), a Fab-scFv-Fc (a molecule in which variable regions of an antibody are replaced with a Fab and an scFv, respectively, and a Fab and an scFv are linked to the Fc region), a Fab-scFv (a molecule in which an scFv is linked to a Fab), and a BiTE (molecules in which two scFvs are linked).

In a preferable embodiment, the isolated short-chain peptide can be a peptide having the amino acid sequence set forth in SEQ ID NO: 50. In a preferable embodiment, the isolated short-chain peptide can be a peptide having the amino acid sequence set forth in SEQ ID NO: 51. In a preferable embodiment, the isolated short-chain peptide can be a peptide having the amino acid sequence set forth in SEQ ID NO: 52. In a preferable embodiment, the isolated short-chain peptide can be a peptide having the amino acid sequence set forth in SEQ ID NO: 53. In a preferable embodiment, the isolated short-chain peptide can be a peptide having the amino acid sequence set forth in SEQ ID NO: 54. In a more preferable embodiment, the antibody of the present invention can bind to one or more of the isolated short-chain peptides and an antibody fragment containing an scFv as a partial structure. Examples of the antibody fragment containing an scFv as a partial structure include any of protein molecules containing an scFv as mentioned above as an antigen-binding portion.

In a preferable embodiment, the isolated short-chain peptide can be a peptide having the amino acid sequence set forth in SEQ ID NO: 55. In a preferable embodiment, the isolated short-chain peptide can be a peptide having the amino acid sequence set forth in SEQ ID NO: 56. In a preferable embodiment, the isolated short-chain peptide can be a peptide having the amino acid sequence set forth in SEQ ID NO: 57. In a preferable embodiment, the isolated short-chain peptide can be a peptide having the amino acid sequence set forth in SEQ ID NO: 58. In a preferable embodiment, the isolated short-chain peptide can be a peptide having the amino acid sequence set forth in SEQ ID NO: 59. In a more preferable embodiment, the antibody of the present invention can bind to one or more of the isolated short-chain peptides and an antibody fragment containing an scFv as a partial structure. Examples of the antibody fragment containing an scFv as a partial structure include any of protein molecules containing an scFv as mentioned above as an antigen-binding portion.

In a preferable embodiment, the isolated short-chain peptide can be a peptide having the amino acid sequence set forth in SEQ ID NO: 60. In a preferable embodiment, the isolated short-chain peptide can be a peptide having the amino acid sequence set forth in SEQ ID NO: 61. In a preferable embodiment, the isolated short-chain peptide can be a peptide having the amino acid sequence set forth in SEQ ID NO: 62. In a preferable embodiment, the isolated short-chain peptide can be a peptide having the amino acid sequence set forth in SEQ ID NO: 63. In a preferable embodiment, the isolated short-chain peptide can be a peptide having the amino acid sequence set forth in SEQ ID NO: 64. In a more preferable embodiment, the antibody of the present invention can bind to one or more of the isolated short-chain peptides and an antibody fragment containing an scFv as a partial structure. Examples of the antibody fragment containing an scFv as a partial structure include any of protein molecules containing an scFv as mentioned above as an antigen-binding portion.

In a preferable embodiment, the isolated short-chain peptide can be a peptide having the amino acid sequence set forth in SEQ ID NO: 65. In a preferable embodiment, the isolated short-chain peptide can be a peptide having the amino acid sequence set forth in SEQ ID NO: 66. In a preferable embodiment, the isolated short-chain peptide can be a peptide having the amino acid sequence set forth in SEQ ID NO: 67. In a preferable embodiment, the isolated short-chain peptide can be a peptide having the amino acid sequence set forth in SEQ ID NO: 68. In a preferable embodiment, the isolated short-chain peptide can be a peptide having the amino acid sequence set forth in SEQ ID NO: 69. In a more preferable embodiment, the antibody of the present invention can bind to one or more of the isolated short-chain peptides and an antibody fragment containing an scFv as a partial structure. Examples of the antibody fragment containing an scFv as a partial structure include any of protein molecules containing an scFv as mentioned above as an antigen-binding portion.

In a preferable embodiment, the isolated short-chain peptide can be a peptide having the amino acid sequence set forth in SEQ ID NO: 70. In a preferable embodiment, the isolated short-chain peptide can be a peptide having the amino acid sequence set forth in SEQ ID NO: 71. In a more preferable embodiment, the antibody of the present invention can bind to one or more of the isolated short-chain peptides and an antibody fragment containing an scFv as a partial structure. Examples of the antibody fragment containing an scFv as a partial structure include any of protein molecules containing an scFv as mentioned above as an antigen-binding portion.

In a preferable embodiment, the isolated short-chain peptide can be a peptide having the amino acid sequence set forth in any one of SEQ ID NOs: 19, 20, 23 and 24.

In a preferable embodiment, the antibody of the present invention can bind to an scFv. In a more preferable embodiment, the antibody of the present invention can bind to an antibody fragment containing an scFv as a partial structure. In a further preferable embodiment, the antibody of the present invention can bind to a CAR.

In a particularly preferable embodiment, the antibody of the present invention binds to a region of 1 to 10 amino acids on the C terminal side of the first region of an scFv. In a particularly preferable embodiment, the antibody of the present invention binds to a region of 1 to 10 amino acids on the N terminal side of the second region of an scFv. Accordingly, in a particularly preferable embodiment, the antibody of the present invention does not bind or binds only with low binding affinity to a peptide containing the sequence of a linker (for example, a whole length thereof) and not containing the amino acid on the C terminal side of the first region of an scFv and a peptide containing the sequence of a linker (for example, a whole length thereof) and not containing the amino acid sequence on the N terminal side of the second region of an scFv. In a particularly preferable embodiment, the antibody of the present invention binds to a linking part of a region of 1 to 10 amino acids on the C terminal side of the first region of an scFv and a linker, and does not bind or binds only with low binding affinity to the linker portion (for example, a whole length thereof). In a particularly preferable embodiment, the antibody of the present invention binds to a linking part of the linker and the second region of scFv and does not bind or bind only with low binding affinity to the linker portion (for example, a whole length thereof). The lower binding affinity may mean that an antibody has a KD value more than 1, 1.1 times or more, 1.2 times or more, 1.3 times or more, 1.4 times or more, 1.5 times or more, 2 times or more, 3 times or more, 4 times or more, 5 times or more, 6 times or more, 7 times or more, 8 times or more, 9 times or more, 10 times or more, 100 times or more, or 1000 times or more as large as the KD value for a peptide containing an amino acid region of 1 to 10 amino acids on the C terminal side of the first region of an scFv continuous to a linker.

In an embodiment, the antibody of the present invention can bind to any one of the peptides having the amino acid sequences of SEQ ID NOs: 13 to 18 and 26 to 29, and can bind to an scFv. In an embodiment, the antibody of the present invention can bind to a peptide having the amino acid sequence of SEQ ID NO: 13 or 14 and can bind to an scFv. In an embodiment, the antibody of the present invention can bind to any one of the peptides having the amino acid sequences of SEQ ID NOs: 15 to 18 and can bind to an scFv. In an embodiment, the antibody of the present invention can bind to any one of the peptides having the amino acid sequences of SEQ ID NOs: 26 to 28 and can bind to an scFv. In an embodiment, the antibody of the present invention can bind to a peptide having the amino acid sequences of SEQ ID NO: 29 and can bind to an scFv. In an embodiment, the ratio of the binding affinity (KD value) for a peptide consisting of a linker is, for example, less than 1, 1/10 or less, 1/100 or less, 1/1,000 or less, 10⁻⁴ times or less, 10⁻⁵ times or less, 10⁻⁶ times or less, 10⁻⁷ times or less, 10⁻⁸ times or less, 10⁻⁹ times or less, 10⁻¹⁰ times or less, 10⁻¹¹ times or less, or 10⁻¹² times or less, compared to the binding affinity (KD value) for the region of 1 to 10 amino acids on the C terminal side of the first region of an scFv or the region of 1 to 10 amino acids on the N terminal side peptide of a second region.

In a preferable embodiment, the antibody of the present invention can bind to an isolated short-chain peptide as mentioned above and an scFv. In a more preferable embodiment, the antibody of the present invention can bind to an isolated short-chain peptide as mentioned above and an antibody fragment (particularly, an scFv portion thereof) containing an scFv as a partial structure. Examples of the antibody fragment containing an scFv as a partial structure include all protein molecules containing an scFv as mentioned above as an antigen-binding portion. In a further preferable embodiment, the antibody of the present invention can bind to an isolated short-chain peptide as mentioned above and a CAR (particularly, an scFv portion thereof).

In an embodiment, the antibody of the present invention may be an antibody that binds to an scFv, wherein:
(i) the antibody contains a heavy-chain variable region containing heavy-chain CDR1 to 3 of a heavy-chain variable region having an amino acid sequence set forth in SEQ ID NO: 91, as heavy-chain CDR1 to 3 thereof, respectively, and a light-chain variable region having light-chain CDR1 to 3 of a light-chain variable region having an amino acid sequence set forth in SEQ ID NO: 95 as light-chain CDR1 to 3 thereof, respectively;
(ii) the antibody contains a heavy-chain variable region containing heavy-chain CDR1 having an amino acid sequence set forth in SEQ ID NO: 92, heavy-chain CDR2 having an amino acid sequence set forth in SEQ ID NO: 93, and heavy-chain CDR3 having an amino acid sequence set forth in SEQ ID NO: 94; and a light-chain variable region containing light-chain CDR1 having an amino acid sequence set forth in SEQ ID NO: 96, light-chain CDR2 having an amino acid sequence set forth in SEQ ID NO: 97, and light-chain CDR3 having an amino acid sequence set forth in SEQ ID NO: 98;
(iii) the antibody contains a heavy-chain variable region containing heavy-chain CDR1 to 3 of a heavy-chain variable region having an amino acid sequence set forth in SEQ ID NO: 99 as heavy-chain CDR1 to 3 thereof, respectively, and a light-chain variable region containing light-chain CDR1 to 3 of a light-chain variable region having an amino acid sequence set forth in SEQ ID NO: 100 as light-chain CDR1 to 3 thereof, respectively;
(iv) the antibody contains a heavy-chain variable region containing heavy-chain CDR1 having an amino acid sequence set forth in SEQ ID NO: 100, heavy-chain CDR2 having an amino acid sequence set forth in SEQ ID NO: 101, and heavy-chain CDR3 having an amino acid sequence set forth in SEQ ID NO: 102, and a light-chain variable region containing light-chain CDR1 having an amino acid sequence set forth in SEQ ID NO: 104, light-chain CDR2 having an amino acid sequence set forth in SEQ ID NO: 105, and light-chain CDR3 having an amino acid sequence set forth in SEQ ID NO: 106;
(v) the antibody contains a heavy-chain variable region containing heavy-chain CDR1 to 3 of a heavy-chain variable region having an amino acid sequence set forth in SEQ ID NO: 121 as heavy-chain CDR1 to 3 thereof, respectively, and a light-chain variable region containing light-chain CDR1 to 3 of a light-chain variable region having an amino acid sequence set forth in SEQ ID NO: 122 as light-chain CDR1 to 3 thereof, respectively;
(vi) the antibody contains a heavy-chain variable region containing heavy-chain CDR1 to 3 of a heavy-chain variable region having an amino acid sequence set forth in SEQ ID NO: 153 as heavy-chain CDR1 to 3 thereof, respectively, and a light-chain variable region containing light-chain CDR1 to 3 of a light-chain variable region having an amino acid sequence set forth in SEQ ID NO: 154 as light-chain CDR1 to 3 thereof, respectively;
(vii) the antibody contains a heavy-chain variable region having an amino acid sequence set forth in SEQ ID NO: 91 and a light-chain variable region having an amino acid sequence set forth in SEQ ID NO: 95;
(viii) the antibody contains a heavy-chain variable region having an amino acid sequence set forth in SEQ ID NO: 99 and a light-chain variable region having an amino acid sequence set forth in SEQ ID NO: 103;
(ix) the antibody contains a heavy-chain variable region having an amino acid sequence set forth in SEQ ID NO: 121 and a light-chain variable region having an amino acid sequence set forth in SEQ ID NO: 122;
(x) the antibody contains a heavy-chain variable region having an amino acid sequence set forth in SEQ ID NO: 153 and a light-chain variable region having an amino acid sequence set forth in SEQ ID NO: 154;
(xi) the antibody competes with the above (vii), (viii), (ix), or (x) for binding to an scFv; or
(xii) the antibody is any one of the antibodies mentioned above, which does not substantially bind to a naturally occurring (*in vivo*) immunoglobulin.

These antibodies may be non-human antibodies and can be preferably humanized antibodies or human antibodies. For example, the antibody to be administered to a human is an antibody set forth in (xii) and preferably a humanized antibody or a human antibody thereof. The antibody to be used *in-vitro* test may be a non-human antibody and may not have the properties set forth in (xii). An antibody that competes with the antibody set forth in (ix) for binding to an scFv can be an antibody that does not substantially bind to a naturally occurring *(in vivo)* immunoglobulin. An antibody that competes with the antibody set forth in (x) for binding to an scFv can be an antibody that does not substantially bind to a naturally occurring (*in vivo)* immunoglobulin. An antibody that competes with the antibody set forth in (ix) for binding to an scFv can be an antibody that does not substantially bind to a VH-L1-VL scFv. An antibody that competes with the antibody set forth in (ix) for binding to an scFv can be an antibody that does not substantially bind to a VH-L1-VL scFv. An antibody that competes with the antibody set forth in (ix) for binding to an scFv can be an antibody that does not substantially bind to a naturally occurring *(in vivo)* immunoglobulin and an antibody that does not substantially bind to a VH-L1-VL scFv. An antibody that competes with the antibody set forth in (x) for binding to an scFv can be an antibody that does not substantially bind to a VH-L1-VL scFv. An antibody that competes with the antibody set forth in (ix) for binding to an scFv can be an antibody that does not substantially bind to a naturally occurring *(in vivo)* immunoglobulin and an antibody that does not substantially bind to a VH-L1-VL scFv.

In an embodiment, the present invention provides an antibody that binds to a VL-L₁-VH scFv, and more specifically,
(xv) an antibody having a heavy-chain variable region containing heavy-chain CDR1 to 3 set forth in SEQ ID NOs: 123 to 125, respectively, and a light-chain variable region having light-chain CDR1 to 3 set forth in SEQ ID NO: 126 to 128, respectively;
(xv-2) an antibody having a heavy-chain variable region containing heavy-chain CDR1 to 3 set forth in SEQ ID NOs: 129 to 131, respectively, and a light-chain variable region having light-chain CDR1 to 3 set forth in SEQ ID NOs: 132 to 134, respectively;
(xv-3) an antibody having a heavy-chain variable region containing heavy-chain CDR1 to 3 set forth in SEQ ID NOs: 135 to 137, respectively, and a light-chain variable region having light-chain CDR1 to 3 set forth in SEQ ID NOs: 138 to 140, respectively;
(xv-4) an antibody having a heavy-chain variable region containing heavy-chain CDR1 to 3 set forth in SEQ ID NOs: 141 to 143, respectively, and a light-chain variable region having light-chain CDR1 to 3 set forth in SEQ ID NOs: 144 to 146, respectively;
(xv-5) an antibody having a heavy-chain variable region containing heavy-chain CDR1 to 3 set forth in SEQ ID NOs: 147 to 149, respectively, and a light-chain variable region having light-chain CDR1 to 3 set forth in SEQ ID NOs: 150 to 152, respectively;
(xvi) an antibody having a heavy-chain variable region containing heavy-chain CDR1 to 3 set forth in SEQ ID NOs: 155 to 157, respectively, and a light-chain variable region having light-chain CDR1 to 3 set forth in SEQ ID NOs: 158 to 160, respectively;
(xvi-2) an antibody having a heavy-chain variable region containing heavy-chain CDR1 to 3 set forth in SEQ ID NOs: 161 to 163, respectively, and a light-chain variable region having light-chain CDR1 to 3 set forth in SEQ ID NOs: 164 to 166, respectively;
(xvi-3) an antibody having a heavy-chain variable region containing heavy-chain CDR1 to 3 set forth in SEQ ID NOs: 167 to 169, respectively, and a light-chain variable region having light-chain CDR1 to 3 set forth in SEQ ID NOs: 170 to 172, respectively;
(xvi-4) an antibody having a heavy-chain variable region containing heavy-chain CDR1 to 3 set forth in SEQ ID NOs: 173 to 175, respectively, and a light-chain variable region having light-chain CDR1 to 3 set forth in SEQ ID NOs: 176 to 178, respectively;
(xvi-5) an antibody having a heavy-chain variable region containing heavy-chain CDR1 to 3 set forth in SEQ ID NOs: 179 to 181, respectively, and a light-chain variable region having light-chain CDR1 to 3 set forth in SEQ ID NOs: 182 to 184, respectively; or
(xvii) any one of the above antibodies that does not substantially bind to a naturally occurring (*in vivo*) immunoglobulin.

These antibodies may be non-human antibodies and can be preferably humanized antibodies or human antibodies. For example, the antibody to be administered to a human is an antibody set forth in (xvii) and preferably a humanized antibody or a human antibody. The antibody to be used in *in-vitro* tests may be a non-human antibody or may not have properties set forth in (xvii). These antibodies are preferably antibodies that do not substantially bind to a naturally occurring (*in vivo*) immunoglobulin. Also, these antibodies are preferably antibodies that do not substantially bind to a VH-L1-VL scFv. These antibodies can be more preferably antibodies that do not substantially bind to a naturally occurring (*in vivo*) immunoglobulin or substantially bind to a VH-L1-VL scFv.

In an embodiment, the present invention provides an antibody that binds to a VL-L₁-VH scFv, and containing
a heavy-chain variable region containing
heavy-chain CDR1, which is a partial sequence of a 6 to 10 amino acid length of the amino acid sequence set forth in SEQ ID NO: 121 and has a sequence containing the amino acid sequence (TSY) set forth in SEQ ID NO: 185,
heavy-chain CDR2, which is a partial sequence of a 5 to 16 amino acid length of the amino acid sequence set forth in SEQ ID NO: 121 and has a sequence containing the amino acid sequence (WNDGD) set forth in SEQ ID NO: 186, and
heavy-chain CDR3, which is a partial sequence of an 8 or 9 amino acid length of the amino acid sequence set forth in SEQ ID NO: 121 and has a sequence containing the amino acid sequence (MNGYKD) set forth in SEQ ID NO: 187; and:
   a light-chain variable region containing
   light-chain CDR1 which is a partial sequence of a 6 to 11 amino acid length of the amino acid sequence set forth in SEQ ID NO: 122 and has a sequence containing the amino acid sequence (KRY) set forth in SEQ ID NO: 188,
   light-chain CDR2 which is a partial sequence of a 3 to 10 amino acid length of the amino acid sequence set forth in SEQ ID NO: 122 and has a sequence containing the amino acid sequence (EDS) set forth in SEQ ID NO: 189, and
   light-chain CDR3 which is a partial sequence of a 10 or 11 amino acid length of the amino acid sequence set forth in SEQ ID NO: 122 and has a sequence containing the amino acid sequence (HSTYSDDKLR) set forth in SEQ ID NO: 190.

These antibodies may be non-human antibodies and can be preferably humanized antibodies or human antibodies. For example, the antibody to be administered to a human is an antibody set forth in (xvii) and preferably a humanized antibody or a human antibody. The antibody to be used in *in-vitro* tests may be a non-human antibody or may not have properties set forth in (xvii). These antibodies are preferably antibodies that do not substantially bind to a naturally occurring (*in vivo*) immunoglobulin. Also, these antibodies are preferably antibodies that do not substantially bind to a VH-L1-VL scFv. Also, these antibodies can be more preferably antibodies that do not substantially bind to a naturally occurring (*in vivo)* immunoglobulin and do not substantially bind to a VH-L1-VL scFv.

In an embodiment, the present invention provides an antibody that binds to a VL-L₁-VH scFv, and containing
a heavy-chain variable region containing
heavy-chain CDR1, which is a partial sequence of a 6 to 8 amino acid length of the amino acid sequence set forth in SEQ ID NO: 153 and has a sequence containing the amino acid sequence (TSY) set forth in SEQ ID NO: 191,
heavy-chain CDR2, which is a partial sequence of a 5 to 16 amino acid length of the amino acid sequence set forth in SEQ ID NO: 153 and has a sequence containing the amino acid sequence (YYDGD) set forth in SEQ ID NO: 192,
heavy-chain CDR3, which is a partial sequence of a 7 to 9 amino acid length of the amino acid sequence set forth in SEQ ID NO: 153 and has a sequence containing the amino acid sequence (SIGAED) set forth in SEQ ID NO: 193; and
a light-chain variable region containing
light-chain CDR1, which is a partial sequence of a 6 to 11 amino acid length of the amino acid sequence set forth in SEQ ID NO: 154 and has a sequence containing the amino acid sequence (KRY) set forth in SEQ ID NO: 194,
light-chain CDR2, which is a partial sequence of a 3 to 10 amino acid length of the amino acid sequence set forth in SEQ ID NO: 154 and has a sequence containing the amino acid sequence (EDS) set forth in SEQ ID NO: 195, and
light-chain CDR3, which is a partial sequence of a 10 or 11 amino acid length of the amino acid sequence set forth in SEQ ID NO: 154 and has a sequence containing the amino acid sequence (HSTYSDDKLR) set forth in SEQ ID NO: 196.

These antibodies may be non-human antibodies and can be preferably humanized antibodies or human antibodies. For example, the antibody to be administered to a human is an antibody set forth in (xvii) and preferably a humanized antibody or a human antibody. The antibody to be used in *in vitro* tests may be a non-human antibody or may not have properties set forth in (xvii). These antibodies are preferably antibodies that do not substantially bind to a naturally occurring (*in vivo*) immunoglobulin. Also, these antibodies are preferably antibodies that do not substantially bind to a VH-L1-VL scFv. Also, these antibodies can be more preferably antibodies that do not substantially bind to a naturally occurring (*in vivo*) immunoglobulin or do not substantially bind to a VH-L1-VL scFv.

A VL-L₁-VH scFv is an scFv containing a VL, a linker, and a VH in this order and the linker has the amino acid sequence of SEQ ID NO: 29. VH-L1-VL scFv is an scFv containing a VH, a linker, and a VL in this order and the linker has the amino acid sequence of SEQ ID NO: 29.

In the above (i), (iii), (v), and (vi), the amino acid sequences of CDRs can be those determined in accordance with, e.g., the Kabat numbering scheme, Chothia, AbM, contact, IMGT, Aho, or Martin (Enhanced Chothia), as mentioned above. In the above (ii), (iv), (xv-2), and (xvi-2), the amino acid sequences of CDRs can be those determined in accordance with, e.g., the Kabat numbering scheme. CDRs determined by the Kabat numbering scheme can be preferably used.

As the antibody of the present invention or an antigen-binding fragment thereof, an antibody or an antigen-binding fragment thereof having a mutation selected from an insertion, deletion, addition and substitution of one to several amino acids in the antibody of the present invention, can be included. In an embodiment, the present invention provides an antibody or an antigen-binding fragment thereof containing at least one, at least 2, at least 3 or more CDRs, which are substantially the same as at least 2, at least 3 or more CDRs of the antibody of the present invention or an antigen-binding fragment thereof. Another embodiment includes an antibody having at least 2, 3, 4, 5 or 6 CDRs, which are substantially the same as in at least 2, 3, 4, 5 or 6 CDRs in the antibody of the present invention or an antigen-binding fragment thereof, or derived from the antibody of the present invention or an antigen-binding fragment thereof. In an embodiment, at least 1, 2, 3, 4, 5, or 6 CDRs, which is at least about 85%, 86%, 87%, 88%, 89%, 90%, 95%, 96%, 97%, 98%, or 99% identical with at least 1, 2, or 3 CDRs in the antibody of the present invention or an antigen-binding fragment thereof, are included. In an embodiment, at least 1, 2, 3, 4, 5, or 6 CDRs contain at least one insertion, deletion, addition or substitution in at least 1, 2, 3, 4, 5, or 6 CDRs of the antibody of the present invention or an antigen-binding fragment thereof or derived from the antibody of the present invention or an antigen-binding fragment thereof. In the antibody of the present invention or an antigen-binding fragment thereof, an antibody or an antigen-binding fragment thereof having an amino acid sequence identity of 80% or more, 85% or more, 90% or more, or 95% or more and an antigen specificity of the antibody, can be included. The antibody of the present invention or an antigen-binding fragment thereof may include an antibody or an antigen-binding fragment thereof having an amino acid sequence identity of 80% or more, 85% or more, 90% or more, or 95% or more in the region except for 6 CDRs and an antigen specificity of the antibody.

### <Short-chain peptide of the present invention>

The present invention may provide an isolated short-chain peptide as mentioned above. The isolated short-chain peptide may have a 10 to 20 amino acid length, more specifically, a 13 to 20 amino acid length. The isolated short-chain peptide may have an amino acid sequence set forth in any one of, for example, SEQ ID NOs: 19 to 24 and 50 to 71. The term "isolated" herein refers to being separated from other regions of an scFv. The term "short chain" means that a chain has a length of, for example, at most 50 amino acids, preferably 30 amino acids or less, and more preferably 20 amino acids or less. In a preferable embodiment, in order to use immunogenicity of an scFv portion in the isolated short-chain peptide, the length of amino acid sequence of the scFv is 1 time or more, 1.1 times or more, 1.2 times or more, 1.3 times or more, 1.4 times or more, 1.5 times or more, 1.6 times or more, 1.7 times or more, 1.8 times or more, 1.9 times or more, 2.0 times or more, 2.1 times or more, 2.2 times or more, 2.3 times or more, 2.4 times or more, 2.5 times or more, 2.6 times or more, 2.7 times or more, 2.8 times or more, 2.9 times or more, or 3 times or more as long as than that of the linker portion.

### <Method for obtaining antibody>

An antibody can be prepared by a method commonly known in the technical field. An antibody can be obtained, for example, by immunizing an animal (for example, a mouse, a rat, or a rabbit) with an isolated peptide as mentioned above. The immunogenicity of an isolated peptide can be enhanced by linking keyhole limpet hemocyanin (KHL) serving as a carrier protein to the peptide. If necessary, booster immunization can be carried out a plurality of times. A cell producing a monoclonal antibody can be obtained by taking an antibody-producing cell from a lymph tissue such as the spleen and lymph node. The cell producing a monoclonal antibody can be obtained by fusing the antibody-producing cell obtained and a myeloma cell to obtain a hybridoma and subjecting the hybridoma to single-cell cloning. A monoclonal antibody can be obtained as a culture supernatant of a hybridoma. The monoclonal antibody obtained can be subjected to a test (for example, ELISA) for examining reactivity to an antigen. The monoclonal antibody can be also subjected to a test (for example, ELISA) for examining reactivity to an scFv or an antigen-binding compound containing an scFv as an antigen binding site. The monoclonal antibody obtained can be also subjected to a test (for example, flow cytometry) for examining reactivity to a CAR-T cell. In this manner, an antibody satisfying at least one of the following (i) to (vi) can be selected or identified from the group of antibodies:
(i) an antibody that binds to an isolated peptide having an amino acid sequence of a region present within the first region and having 1 to 10 amino acids from the C terminal of the first region, and a partial region of a linker continuous with the region and having 1 to 10 amino acids from the N terminal of the linker, the isolated peptide having 13 to 20 amino acid length;
(ii) an antibody that binds to an isolated peptide having an amino acid sequence of a partial region of a linker continuous with the second region and having 1 to 10 amino acids from the C terminal of the linker, and a region present within the second region and having 1 to 10 amino acids from the N terminal of the second region, and the isolated peptide having 13 to 20 amino acid length;
(iii) an antibody that binds to an isolated peptide having an amino acid sequence set forth in any one of SEQ ID NOs: 19, 20, 23 and 24;
(iv) an antibody that binds to an isolated peptide having an amino acid sequence set forth in any one of SEQ ID NOs: 19 to 24 and 50 to 71;
(v) an antibody that binds to a region present within the first region and having 1 to 10 amino acids from the C terminal of the first region and a partial region of a linker continuous with the region and having 1 to 10 amino acids from the N terminal of the linker; and
(vi) an antibody that binds to a partial region of a linker continuous with the second region and having 1 to 10 amino acids from the C terminal of the linker, and a region present within the second region and having 1 to 10 amino acids from the N terminal of the second region.

In this manner, an antibody that binds to an scFv can be selected. In an embodiment, the group of antibodies can be a group of antibodies that bind to an scFv. In a preferable embodiment, the group of antibodies can be a group of antibodies that bind to any one of the isolated short-chain peptides mentioned above. In more preferable embodiment, the group of antibodies can be a group of antibodies that bind to any one of the isolated short-chain peptides mentioned above, not binding or binding only with low binding affinity to a linker portion (for example, whole length thereof).

In an embodiment, there is provided a method for selecting or identifying, from the group of antibodies, an antibody that competes, for binding to an scFv, with any one of
(b-iii) an antibody containing a heavy-chain variable region having an amino acid sequence set forth in SEQ ID NO: 91 and a light-chain variable region having an amino acid sequence set forth in SEQ ID NO: 95;
(b-iv) an antibody containing a heavy-chain variable region having an amino acid sequence set forth in SEQ ID NO: 99 and a light-chain variable region having an amino acid sequence set forth in SEQ ID NO: 103;
(b-xi) an antibody containing a heavy-chain variable region having an amino acid sequence set forth in SEQ ID NO: 121 and a light-chain variable region having an amino acid sequence set forth in SEQ ID NO: 122; and
(b-xii) an antibody containing a heavy-chain variable region having an amino acid sequence set forth in SEQ ID NO: 153 and a light-chain variable region having an amino acid sequence set forth in SEQ ID NO: 154, and that binds to an scFv.

In an embodiment, the group of antibodies can be, for example, a group of antibodies that bind to an scFv. In an embodiment, the group of antibodies can be a group of antibodies satisfying one or more selected from
(i) an antibody that binds to an isolated peptide having an amino acid sequence of a region present within the first region and having 1 to 10 amino acids from the C terminal of the first region, and a partial region of a linker continuous with the region and having 1 to 10 amino acids from the N terminal of the linker, the isolated peptide having 13 to 20 amino acid length;
(ii) an antibody that binds to an isolated peptide having an amino acid sequence of a partial region of a linker continuous with the second region and having 1 to 10 amino acids from the C terminal of the linker, and a region present within the second region and having 1 to 10 amino acids from the N terminal of the second region, the isolated peptide having 13 to 20 amino acid length;
(iii) an antibody that binds to an isolated peptide having an amino acid sequence set forth in any one of SEQ ID NOs: 19, 20, 23 and 24;
(iv) an antibody that binds to an isolated peptide having an amino acid sequence set forth in any one of SEQ ID NOs: 19 to 24 and 50 to 71;
(v) an antibody that binds to a region present within the first region and having 1 to 10 amino acids from the C terminal of the first region and a partial region of a linker continuous with the region and having 1 to 10 amino acids from the N terminal of the linker; and
(vi) an antibody that binds to a partial region of a linker continuous with the second region and having 1 to 10 amino acids from the C terminal of the linker, and a region present within the second region and having 1 to 10 amino acids from the N terminal of the second region, and
may further satisfy a condition of binding to an scFv.

In an embodiment, the group of antibodies can be a group of antibodies that (iii) bind to an isolated peptide having an amino acid sequence set forth in any one of SEQ ID NOs: 19, 20, 23 and 24; or (iv) bind to an isolated peptide having an amino acid sequence set forth in any one of SEQ ID NOs: 19 to 24 and 50 to 71.

In an embodiment, the above antibody
(vii) does not exhibit significant binding affinity for an scFv in which the order of a heavy-chain variable region and a light-chain variable region is interchanged.

In an embodiment, the above antibody
(viii) exhibits significant binding affinity even for an scFv in which the order of the heavy-chain variable region and the light-chain variable region is interchanged.

The binding affinity of an antibody to a peptide or an scFv can be determined, for example, by a known method in the technical field such as ELISA. The fact that an antibody (vii) does not exhibit significant binding affinity for an scFv in which the order of a heavy-chain variable region and a light-chain variable region is interchanged, can be determined by applying ELISA to the scFv and its original scFv in which the order of a heavy-chain variable region and a light-chain variable region was not interchanged. The fact that an antibody does not bind or binds only with low binding affinity to a linker portion (for example, whole length thereof) can be determined by subjecting the peptide of the linker portion to ELISA and compared to the results of ELISA of any one of the above isolated peptides. As mentioned above, in this disclosure, an appropriate assay system employed based on the binding property, and an antibody can be selected by the appropriate assay system.

The nucleic acid sequences encoding a heavy-chain and a light-chain of the antibody selected or identified and the amino acid sequences thereof can be estimated. The nucleic acid sequences encoding a heavy-chain and a light-chain of the antibody can be estimated, for example, by obtaining mRNAs encoding the heavy-chain and the light-chain of the antibody from a cell producing the antibody, synthesizing cDNAs and determining the sequences of cDNA. Based on the nucleic acid sequences thus estimated, the amino acid sequences of the heavy-chain and the light-chain of the antibody can be estimated. Based on the amino acid sequences of a heavy-chain and a light-chain estimated, the amino acid sequences of a heavy-chain variable region and a light-chain variable region can be estimated. Based on the amino acid sequences of a heavy-chain variable region and a light-chain variable region thus estimated, amino acid sequences of heavy-chain CDR1 to 3 and light-chain CDR1 to 3 can be estimated. The antibody can be produced from an antibody-producing cell (for example, Chinese hamster ovary cell (CHO)) by a genetic recombination technology routinely used in the technical field. Accordingly, the method of the present invention may further include obtaining an antibody containing heavy-chain variable region containing heavy-chain CDR1 to 3 and light-chain variable region having light-chain CDR1 to 3 from an antibody-producing cell containing a gene encoding the identified antibody containing a heavy-chain variable region containing heavy-chain CDR1 to 3 and a light-chain variable region having light-chain CDR1 to 3 of the antibody identified.

### <Method for targeting or detecting cell>

The present invention provides a method for detecting a cell. The cell to be detected can be a cell expressing a chimeric antigen receptor (CAR) containing an scFv. In an embodiment, detection can be made using the antibody of the present invention. Specifically, the method of the present invention may include bringing the antibody of the present invention into contact with a cell that expresses or conceivably expresses a chimeric antigen receptor (CAR) containing an scFv to form a complex of the antibody and the cell. The complex can be detected using, e.g. a label attached to an antibody and detection can be made by flow cytometry.

According to the present invention, a cell expressing a chimeric antigen receptor (CAR) can be an immune cell. The immune cell can be preferably, a T cell or an NK cell. In a preferable embodiment, the T cell expressing a chimeric antigen receptor (CAR) can be a CAR-T cell.

The present invention provides a kit or test agent for detecting an scFv. According to an embodiment, the present invention can provide a kit or test agent containing the antibody of the present invention for detecting a cell expressing a chimeric antigen receptor (CAR) containing an scFv. The kit or test agent may contain a solution containing an antibody. The kit or test agent may contain a solution containing an antibody and reagents for detecting an scFv (for example, a labeled secondary antibody, a reaction buffer, a blocking liquid, and a washing liquid). The kit or test agent may contain a solution containing an antibody and an instruction for detecting an scFv.

In the present invention, an antigen of an scFv can be an antigen expressed on a cancer cell (for example, cancer antigen). The antigen of an scFv can be one or more selected from the group consisting of CD16, CD19, CD20, CD22, CD123, CD171, epidermal growth factor receptor (EGFR, particularly EGFRvIII, type-3 EGFR, de2-7EGFR and HER2), carcinoembryonic antigen (CEA), prostate stem cell antigen (PSCA), B cell maturation antigen (BCMA), CS1, NKG2D, NKp30, B7H6, MUC-16 (CA125), or phan receptors for receptor tyrosine kinases 1 (ROR-1), GD3, GM2, glypican-3 (GPC3), mesothelin, IL13R, c-KIT, c-MET, NY-ESO-1, WT1, MAGE-A3, MAGE-A4, MAGE-A10, E6 of HPV, E7 of HPV, CMV, AFP, FRAME, SSX2, KRAS, HER2, and PD-L1. The antigen of an scFv may be, for example, a protein tag. The antigen of an scFv can be, for example, a fluorescent protein such as fluorescein isothiocyanate (FITC).

In the present invention, a CAR-expressing cell can express a cytokine in addition to a CAR. The CAR-expressing cell may express, for example, interleukin-7 (IL-7) and CCL19. In a preferable embodiment, the CAR-expressing cell can be a CAR-expressing T cell. In the present invention, the CAR-expressing cell may contain a gene encoding thymidine kinase (for example, thymidine kinase of herpes simplex virus) for use in, e.g., removing cells. A cell expressing thymidine kinase takes up ganciclovir and phosphorylates it to induce strong toxicity, and then, it can kill the cell expressing thymidine kinase. A CAR-expressing cell can be removed or regulated by this mechanism.

The present invention provides a method for targeting a cell or an antibody *in vitro* or *in vivo.* The cell to be targeted can be a cell expressing a chimeric antigen receptor (CAR) containing an scFv. In an embodiment, targeting a cell is performed by using the antibody of the present invention. Specifically, the method of the present invention can be carried out in a subject administered with a cell expressing a chimeric antigen receptor (CAR) containing an scFv. The subject may have a cell expressing a chimeric antigen receptor (CAR) containing an scFv, within the body. Accordingly, the cell in the body of the subject can be targeted by administering the antibody of the present invention to the subject. The antibody of the present invention can regulate the function of the cell to be targeted. For example, the antibody of the present invention inhibits the binding between a CAR-T cell and a target antigen to impair the function of the CAR-T cell.

The antibody to be targeted may be an antibody containing one or more of the isolated single-chain peptides mentioned above and an scFv as a partial structure, or a fragment of the antibody. In an embodiment, an antibody can be targeted by use of the antibody of the present invention. Specifically, the method of the present invention can be carried out in a subject to which an antibody to be targeted is administered. It is possible to target an antibody or a fragment thereof containing one or more of the isolated single-chain peptides mentioned above and an scFv as a partial structure can be targeted by administering the antibody of the present invention to the subject. The antibody of the present invention can regulate the function of the antibody to be targeted. For example, the antibody of the present invention inhibits binding to the antibody to be targeted and an antigen thereof, thereby impairing the function of the antibody to be targeted.

When the antibody of the present invention is used for targeting a cell or an antibody, if a medicine or a reagent is linked to the antibody of the present invention, the medicine or reagent can be delivered to a target cell or a target antibody or a therapeutic target. Examples of the medicine and reagent to be linked to the antibody of the present invention include, but are not particularly limited to, a compound increasing or decreasing the function of the target cell or target antibody, itself (for example, cell activator), a compound therapeutically acting on a target cell or antibody, a compound detectably labeling a target cell or antibody or a therapeutic target of them.

The present invention provides an antibody formulation containing the antibody of the present invention for use in targeting a cell expressing a chimeric antigen receptor (CAR) containing an scFv in a subject to which the cell expressing the chimeric antigen receptor (CAR) containing an scFv is administered.

### Examples

### Example 1: Preparation of antibody that binds to linker region of scFv

In this Example, antibodies that bind to the linker region of an scFv was tried to be prepared. Specifically, in an scFv containing a first region, a linker region and a second region in the order shown in Figure 1, an antibody against an isolated peptide positioned in the region covering the first region and the linker region was obtained. As the first region and the second region, a heavy-chain variable region (VH) and a light-chain variable region (VL) of an antibody were used, respectively, or a light-chain variable region (VL) and a heavy-chain variable region (VH) were used, respectively.

In the scFv, the first region and the second region, or the second region and the first region were designed based on the heavy-chain variable region (VH) and light-chain variable region (VL) of an isotype control IgG. As the linker, a linker having the amino acid sequence set forth in SEQ ID NO: 27 (GGGGSGGGGSGGGGS) or SEQ ID NO: 29 (SSADDAKKDAAKKDDAKKDDAKKDG) was used.

In the following experiment, VH-L₂-VL indicates an scFv containing a VH, a linker, and a VL in this order and the linker has the scFv of SEQ ID NO: 29. VL-L₂-VH indicates an scFv containing, a VL, a linker, and a VH in this order and the linker has the scFv of SEQ ID NO: 29. VH-L₂-VL indicates an scFv containing, a VH, a linker, and VL in this order and the linker has the scFv of SEQ ID NO: 27. VL-L₂-VH indicates an scFv containing a VL, a linker, and a VH in this order and the linker has the scFv of SEQ ID NO: 27.

It is not easy to obtain an antibody against a linker. According to US2019/0092818A, the amino acid sequence of a linker is modified in order to easily obtain an antibody, and then, an antibody that binds to the linker having the modified amino acid sequence is obtained. In this Example, it is intended to obtain a more versatile antibody by using an existing linker sequence as it is. For this purpose, an antibody was tried to be obtained by immunization with a polypeptide having the amino acid sequence of a part spanning the first region and the linker. Specifically, the sequence of a linker is linked to a sequence consisting of 4 or 5 amino acids on the C terminal side of the first region to obtain 4 types of peptides and individually synthesized. The sequences of the isolated peptides used as an immunogen are shown below.

### | [Formula 1]

### Sequence·of·peptide·used·as·immunogen

Peptide·1·(SEQ·ID·NO·22)**: GG VTVSS *GGGGSG* C**
Peptide·2·(SEQ·ID·NO·71)**: GG LEIK *GGGGSG* C**
Peptide·3·(SEQ·ID·NO·21)**: GG VTVSS *SSADDAKK* C**
Peptide·4·(SEQ·ID·NO·70)**: GG LEIK *SSADDAKK* C**

In peptides 1 and 3 in the above, the sequences of the regions consisting of 5 amino acids from the C terminal of a heavy-chain variable region are underlined. In peptides 2 and 4, the regions consisting of 4 amino acids from the C terminal of a light-chain variable region are underlined. In peptides 1 and 2, 6 amino acids on the N terminal side of a linker of SEQ ID NO: 27 are shown in italics. In the above peptides 3 and 4, 8 amino acids on the N terminal side of a linker of SEQ ID NO: 29 are shown in italics. In peptides 1 to 4, GG was added to the N terminal, and C was added to the C terminal. The N terminals of these amino acids were acetylated.

Peptides 1 to 4 were chemically synthesized, and thereafter, keyhole limpet hemocyanin (KHL) serving as a carrier protein was conjugated to each of the peptides via C added to the C terminal, in accordance with a common procedure. Rats were immunized with the obtained conjugates through the footpad and underwent booster immunization through the root of the tail. Lymphocytes were isolated from the iliac lymph nodes and subjected to cell fusion with myeloma cells. The resultant hybridoma cells were subjected to cloning. The cloning was carried out in accordance with the methylcellulose method by picking up a single colony. The obtained hybridoma cells were seeded in the wells of a 96 well plate and cultured while exchanging the medium in the middle. The culture supernatants of the individual clones were examined for binding to an scFv. The supernatants of the clones obtained by immunization with Peptide 1, Peptide 2, Peptide 3, and Peptide 4 were contacted with HEK293T cells temporarily expressing scFv (VH-L₂-VL) plasmid (#530), scFv (VL-L₂-VL) plasmid (#350), scFv (VH-L₁-VL) plasmid (#602), and scFv (VL-L₁-VH) plasmid (#231), respectively, to examine the presence or absence of binding. As a negative control, 293T cells not transfected with the above plasmids were used. As a positive control of an antibody for clones immunized with Peptides 1 to 3, biotinylated protein L was used, whereas FITC-DEX was used as the positive control for a clone immunized with Peptide 4.

From the antibodies produced by the hybridoma obtained by immunizing with Peptide 4, 2D12, 4B12, and 3A4 having particularly high binding affinity to an scFv were selected. The measurement results of the binding affinity were as shown in Table 3. In Table 3, higher numerical values mean stronger binding affinity.

**[Table 3]**

| Table 3: Results of screening using culture supernatant | | |
|---|---|---|
| Name of clone | Positive (%) | |
| | VH25VL (#602) | Transfection (-) |
| 2D12 | 78.8 | 3.9 |
| 3A4 | 86.1 | 46.9 |
| 4B12 | 76.6 | 4.4 |
| Biotin-Protein L | 49.8 | 3.1 |
| Primary antibody (-) PE-anti-rat antibody (+) | 1.6 | 0.7 |

| | | |
|---|---|---|
| * VH-L₁-VL is scFv having VH, linker and VL linked in this order | | |

As mentioned above, antibodies that bind to an scFv were able to be obtained using an isolated peptide designed in the border region between the first region and a linker, as an immunogen. The antibodies bind to an isolated peptide (Peptide 3) used as an immunogen and, needless to say, bind to an scFv.

Clones 2D12, 4B12, and 3A4 selected were subjected to single-cell cloning and the reactivity to an scFv was confirmed once again.

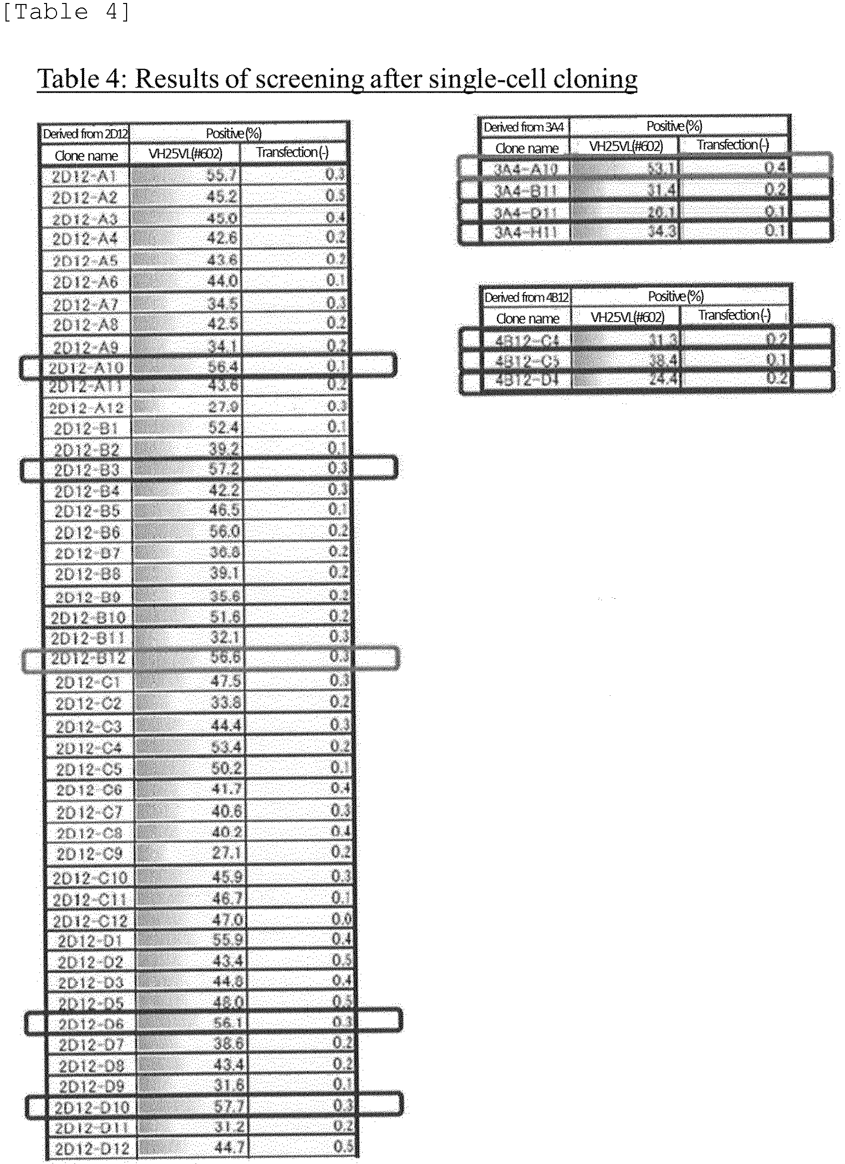

D10, B3, B12, A10, and D6 were obtained from 2D12 by single-cell cloning. A10, H11, B11, and D11 were obtained from 3A4 by single-cell cloning. C5, C4, and D4 were obtained from 4B12. These clones all produced antibodies that bind to an scFv, as shown in Table 4. The reactivities of the antibodies produced from these clones with CAR-T cells containing scFvs (VH-L₁-VL and VH-L₂-VL) were examined. The CARs used herein bind mutually different antigens.

The CARs used herein were as listed in Table 5.

**[Table 5]**

| Table 5:·CAR-T·cells·used·for·examining·reactivity·with·linker-antibody | | |
|---|---|---|
| No. | Form | Structure·of·CAR-T |
| S#067 | VL-L₁-VH | Human·2nd-generation (4-1BB) _hIL7_hCCL19_HSVtk |
| S#075 | VL-L₁-VH | Human·2nd-generation (4-1BB) _hIL7_hCCL19_HSVtk |
| #231 | VL-L₁-VH | Human·3rd-generation |
| S#031 | VH-L₁VL | Human·3rd-generation_hIL7_hCCL19_HSVtk |
| #673 | VH-L₁-VL | Human·3rd-generation |
| S#017 | VH-L₂-VL | Human·3rd-generation_hIL7_hCCL19_HSVtk |
| #530 | VH-L₂-VL | Human·3rd-generation |

The human second generation (4-1BB) herein refers to a molecule in which an scFv region, a CD8α transmembrane domain, 4-1BB domain and CD3ζ domain, all derived from human, are linked in this order. The human third generation herein refers to a molecule in which an scFv region, a CD8α transmembrane domain, CD28 domain, 4-1BB domain and CD3ζ domain, all derived from human, are linked in this order. hIL7_hCCL19_HSVtk indicates a cell expressing human IL-17 and human CCL19 and having thymidine kinase gene of a herpes simplex virus as a suicide gene. The suicide gene is activated by ganciclovir to kill or damage CAR expressing cells.

The C terminal sequences of the first-region of an scFv region that CAR-T shown in Table 5 has (the region closer to the C terminal than CDR3) are listed in Table 6.

**[Table 6]**

| Table·6:·C-terminal·amino·acid·sequence·of·the·first·region·of· scFv·region·that·CAR-T·has | | |
|---|---|---|
| No. | Amino·acid·sequence | SEQ·ID·NO: |
| S#067 | FGGGTKLEIK | 72 |
| S#075 | FGGGTKVEIK | 73 |
| #231 | FGGGTKLEIK | 74 |
| S#031 | GQGTTVTVSS | 75 |
| #673 | GQGTLVTVSA | 76 |
| S#017 | WGQGTTLTVS | 77 |
| #530 | GQGTLVTVSS | 78 |

The results were as shown in Figure 2. As shown in Figure 2, clones B3, B12 and A10 derived from 2D12 and clone A10 derived from 3A4 exhibited reactivity to CAR-T cell (S#031) containing a VH-L₁-VL scFv to antigen D. These clones exhibited reactivity even to CAR-T cell (#673) containing a VH-L₁-VL scFv to antigen E. Note that, none of the antibodies exhibited significant binding affinity for VH-L₂-VL scFvs (negative control, #530) having a linker having a different amino acid sequence. From the results, it was considered that each of the obtained antibodies may bind to a linker or a boundary region between a VH and the linker.

Furthermore, the reactivity between the antibodies produced by the clones mentioned above and CAR-T cells (S#067, S#075, and #231) containing different VL-L₁-VH scFvs, was examined. The results were as shown in Figure 3. As shown in Figure 3, all clones exhibited reactivity to a VL-L₁-VH scFv and CAR-T cells containing this. Further, 3A4-A10 was further subjected to single cloning to obtain three clones. The binding abilities of them to CAR-T cells expressing an scFv were evaluated. The results are shown in Figure 4. All of the clones sufficiently bound to a VH-L₁-VL scFv but did not show a significant reactivity to the CAR-T cells containing the VH-L₂-VL scFv. From these results, it was considered that the obtained antibodies principally bind to a linker.

The binding ability of the antibody obtained by immunization with Peptide 4 to a VL-L₁-VH scFv was examined. From the obtained antibodies produced by hybridomas, 1H5, 2C7, 3E7, and 4B9 exhibiting particularly high binding affinity to an scFv, were selected. The measurement results of binding affinity were shown in Table 7. In Table 7, higher numerical values mean stronger binding affinity.

**[Table 7]**

| Table·7:Results·of·screening·using·culture·supernatant | | |
|---|---|---|
| Name·of·clone | Positive (%) | |
| | VL25VH (#231) | Transfection (-) |
| 1H5 | 52.30 | 0.21 |
| 2C7 | 47.60 | 0.24 |
| 3E7 | 53.90 | 0.24 |
| 4B9 | 53.80 | 0.24 |
| FITC-DEX | 48.90 | 0.74 |
| Primary·antibody·(-)·PE-anti-rat·antibody·(+) | 0.23 | 0.22 |

Using isolated peptides designed in the boundary region between a first region and a linker as immunogens, antibodies that bind to an scFv were obtained. These antibodies bind to an isolated peptide (Peptide 4) used as an immunogen and, needless to say, bind even to an scFv.

Table 8 shows the reactivities to an scFv of 3E7 and 4B9 after single-cell cloning.

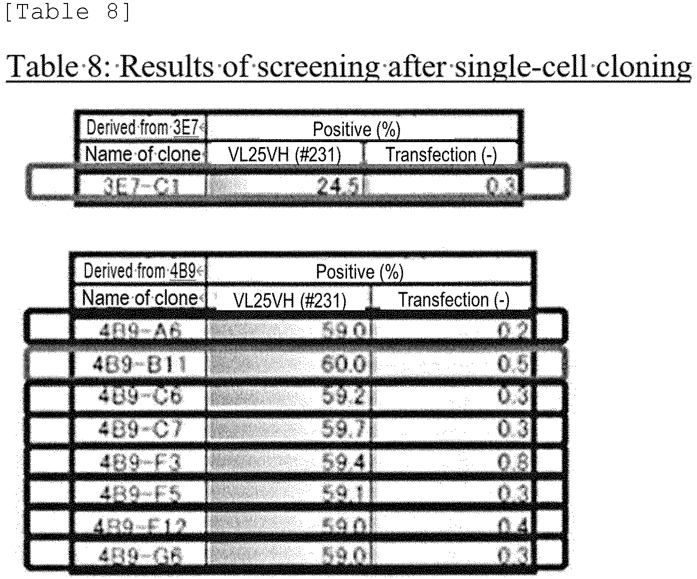

The representative results are shown in Figure 5. As shown in Figure 5, the clones examined above exhibited binding ability to a CAR having a VL-L₁-VH scFv, whereas the binding ability of them to a CAR having a VH-L₁-VL scFv was only low. From this, it was considered that these clones bind to a region spanning from a first region to a linker.

Table 9 shows the reactivities (%) of individual clones to the CAR of individual CAR-T cells. The reactivity was confirmed by detecting the antibody bound to a CAR by a PE-labeled secondary antibody. The reactivity was calculated as the ratio of the number of PE-positive cells to the total number of cells.

**[Table 9]**

| Table·9:·Reactivity ·(%)·of·antibody | | | | |
|---|---|---|---|---|
| | Anti-VL-L₁₋VH scFv antibody | | Anti-VH-L₁-VL scFv antibody | |
| | 3E7-C1 | 4B9-B11 | 2D12-B12 | 3A4-A10-7 |
| VL-L₁-VH CAR-T | | | | |
| S#067 | 41.0 | 41.5 | 34.9 | 22.3 |
| S#075 | 25.9 | 35.7 | 15.0 | 12.3 |
| S#104 | 21.5 | 29.1 | 13.4 | 5.2 |
| #231 | 69.6 | 74.6 | 67.4 | 23.9 |
| S#085 | 41.4 | 49.5 | 0.8 | 2.2 |
| VH-L₁-VL CAR-T | | | | |
| S#050 | 0.1 | 1.1 | 14.5 | 18.5 |
| S#027 | 0.0 | 1.0 | 28.4 | 45.7 |
| S#031 | 2.2 | 9.7 | 64.6 | 45.3 |
| #264 | 0.0 | 0.0 | 11.1 | 36.7 |
| VL-L₂-VH CAR-T | | | | |
| #371 | 0.0 | 0.1 | 0.2 | 0.2 |
| S#066 | 0.1 | 0.1 | 0.9 | 0.2 |
| S#084 | 0.0 | 0.1 | 0.3 | 0.1 |
| S#074 | 0.1 | 1.4 | 0.8 | 0.2 |
| S#097 | 0.0 | 0.1 | 0.3 | 0.1 |
| VH-L₂-VL CAR-T | | | | |
| S#076 | 0.1 | 0.2 | 0.2 | 0.2 |
| #628 | 0.0 | 0.2 | 0.2 | 0.1 |
| S#017 | 0.0 | 0.0 | 0.0 | 0.0 |
| #651 | 0.1 | 0.2 | 0.4 | 0.2 |

With respect to CAR-Ts shown in Table 9 but not shown in Table 6, the sequences of the C terminal (the region closer to the C terminal than CDR3) of the first regions of scFv regions that the CAR-Ts have, are shown in Table 10.
[Table 10]

**Table·10**

| No. | Amino·acid·sequence | SEQ·ID·NO: |
|---|---|---|
| S#104 | FGGGTKVEIK | 107 |
| S#085 | FGGGTKLEIK | 108 |
| S#050 | GQGTLVTVSS | 109 |
| S#027 | GAGTTVTVSS | 110 |
| #264 | GQGTLVTVSA | 111 |
| #371 | FGGGTKVEIK | 112 |
| S#066 | FGGGTKLEIK | 113 |
| S#084 | FGGGTKLEIK | 114 |
| S#074 | FGGGTKLEIK | 115 |
| S#097 | FGGGTKVEIK | 116 |
| S#076 | GQGTLVTVSS | 117 |
| #628 | GQGTTVTVSSGILGS | 118 |
| #651 | GQGTLVTVSS | 119 |

As shown in Table 9, (i) a monoclonal antibody prepared against an invariable region of an scFv variable region and a linker was able to bind to the scFv, regardless of the amino acid sequences of the CDR regions of the scFv. Accordingly, the monoclonal antibody prepared can be an antibody that can detect a wide variety of scFvs having the same sequence regardless of the amino acid sequences of the CDR regions of the scFv. Furthermore, as shown in Table 9, (ii) 3E7-C1 and 4B9-B11 clones bound to VL-L₁-VH but not to an scFv in which the arrangement of VH and VL is interchanged. This result means that these antibodies bind to the region spanning from VH/VL to the linker. Such an antibody can be an antibody detecting a wide variety of scFvs having the same sequence regardless of the amino acid sequences of the CDR regions of the scFv. Antibodies that compete with these antibodies for binding to the same site can be an antibody for detecting a wide variety of scFvs having the same sequence regardless of the amino acid sequences of the CDR regions of the scFv. Furthermore, these antibodies that bind to the region spanning VH/VL and a linker can be useful since they specifically detect the expression of a desired CAR without non-specifically detecting immunoglobulins in a system.

In contrast, 2D12-B12 and 3A4-A10-7 clones bound to an scFv in which the arrangement of VH and VL is interchanged, but not to an scFv in which the linker is replaced with another linker. This result shows that these clones are antibodies that bind to principally to a linker. It is not easy to obtain an antibody that binds to a linker. However, in the present invention, it was suggested that an antibody binding principally to the linker was successfully and efficiently obtained by using a fusion peptide with a partial peptide VH/VL to improve immunogenicity of a linker portion. Such an antibody can detect a wide variety of scFvs regardless of the amino acid sequences of CDR regions of the scFv and the order of VH and VL.

Accordingly, it was demonstrated that the antibody of the present invention is useful in that the antibody can detect an scFv and a CAR-expressing cell containing an scFv, regardless of the amino acid sequences of CDR regions of the scFv (regardless of the antigen).

Moreover, according to Table 9, antibodies against VH-L₁-VL and VL-L₂-VH scFvs showed little reactivity to VH-L₂-VL and VL-L₂-VH scFvs having a different linker. In contrast, the antibody prepared against a VL-L₂-VH scFv showed strong reactivity to CAR-T cells expressing CAR having the VL-L₂-VH scFv. Also, the antibody prepared against a VH-L₂-VL scFv showed strong reactivity to CAR-T cells expressing CAR having the VH-L₂-VL scFv.

Based on the fact that the antibody against a VL-L₁-VH scFv showed little reactivity to a VH-L₂-VL scFv, it is considered that the antibody is not an antibody binding only to a linker or a VL, but to a linking part of the VL and the linker (particularly, to the region spanning from the VL and the linker). As mentioned above, if a linking part of a linker and a VH or a VL is used as an immunogen, it is possible to obtain an antibody against the linking part (or an antibody that binds to a region spanning from a VL and the linker). The antibody against such a linking part showed significant reactivity to a wide variety of scFvs. Furthermore, an antibody that binds to a VH part or a VL part and an antibody that binds to a linker can be useful.

cDNA was synthesized from mRNA encoding a heavy-chain variable region and a light-chain variable region of 2D12-B12 antibody and the amino acid sequences of a heavy-chain variable region and a light-chain variable region were estimated. As a result, it was estimated that the heavy-chain variable region has the amino acid sequence of SEQ ID NO: 91 and the light-chain variable region has the amino acid sequence of SEQ ID NO: 95. The amino acid sequences of heavy-chain CDR1 to 3 (HCDR1 to 3) and light-chain CDR1 to 3 (LCDR1 to 3) were estimated by the Kabat numbering scheme (Kabat, E.A. et al., Sequences of Proteins of Immunological Interest, 5th ed., 1991, Bethesda: US Dept. of Health and Human Services, PHS, NIH.). As a result, HCDR1 to 3 were estimated to have the amino acid sequence of SEQ ID NOs: 92 to 94 and LCDR1 to 3 have the amino acid sequence of SEQ ID NO: 96 to 98.

cDNA was synthesized from mRNA encoding a heavy-chain variable region and a light-chain variable region of 3A4-A10-7 and the amino acid sequences of the heavy-chain variable region and the light-chain variable region were estimated. As a result, it was estimated that the heavy-chain variable region has the amino acid sequence of SEQ ID NO: 99 and the light-chain variable region has the amino acid sequence of SEQ ID NO: 103. The amino acid sequences of heavy-chain CDR1 to 3 (HCDR1 to 3) and light-chain CDR1 to 3 (LCDR1 to 3) were estimated by the Kabat numbering scheme. As a result, HCDR1 to 3 were estimated to have the amino acid sequence of SEQ ID NOs: 100 to 102 and LCDR1 to 3 have the amino acid sequence of SEQ ID NO: 104 to 106.

cDNA was synthesized from mRNA encoding a heavy-chain variable region and a light-chain variable region of 3E7-C1 and the amino acid sequences of the heavy-chain variable region and the light-chain variable region were estimated. As a result, it was estimated that the heavy-chain variable region has the amino acid sequence of SEQ ID NO: 121 and the light-chain variable region has the amino acid sequence of SEQ ID NO: 122. When the amino acid sequences of heavy-chain CDR1 to 3 (HCDR1 to 3) and light-chain CDR1 to 3 (LCDR1 to 3) were estimated, HCDR1 to 3 and LCDR1 to 3 were estimated to have the amino acid sequences of SEQ ID NOs: 123 to 125 and SEQ ID NOs: 126 to 128, respectively according to IMGT; SEQ ID NOs: 129 to 131 and SEQ ID NOs: 132 to 134, respectively according to Kabat; SEQ ID NOs: 135 to 137 and SEQ ID NOs: 138 to 140, respectively according to Chothia; SEQ ID NOs: 141 to 143 and SEQ ID NOs: 144 to 146, respectively according to Contact; and SEQ ID NOs: 147 to 149 and SEQ ID NOs: 150 to 152 respectively according to AbM. Any one of the sets of HCDR1 to 3 and LCDR1 to 3 can be estimated as the CDRs of 3E7-C1. Those skilled in the art can appropriately select CDRs to prepare an antibody having a similar binding property as in 3E7-C1. For example, an antibody having a similar binding property as in 3E7-C1 can be prepared based on the Kabat numbering scheme.

cDNA was synthesized from mRNA encoding a heavy-chain variable region and a light-chain variable region of 4B9-B11 and the amino acid sequences of a heavy-chain variable region and a light-chain variable region were estimated. As a result, it was estimated that the heavy-chain variable region has the amino acid sequence of SEQ ID NO: 153 and the light-chain variable region has the amino acid sequence of SEQ ID NO: 154.
When the amino acid sequences of heavy-chain CDR1 to 3 (HCDR1 to 3) and light-chain CDR1 to 3 (LCDR1 to 3) were estimated, HCDR1 to 3 and LCDR1 to 3 are estimated to have the amino acid sequences of SEQ ID NOs: 155 to 157 and SEQ ID NOs: 158 to 160, respectively according to IMGT; SEQ ID NOs: 161 to 163 and SEQ ID NOs: 164 to 166, respectively according to Kabat; SEQ ID NOs: 167 to 169 and SEQ ID NOs: 170 to 172, respectively according to Chothia; SEQ ID NOs: 173 to 175 and SEQ ID NOs: 176 to 178, respectively according to Contact; and SEQ ID NOs: 179 to 181 and SEQ ID NOs: 182 to 184, respectively according to AbM. Any one of the sets of HCDR1 to 3 and LCDR1 to 3 can be estimated as CDRs of 4B9-B11. Those skilled in the art can appropriately select CDRs to prepare an antibody having a similar binding property as in 4B9-B11. For example, an antibody having a similar binding property as in 4B9-B11 can be prepared based on the Kabat numbering scheme.

**[Table 11-1]**

| Table 11: Sequence listing | | |
|---|---|---|
| SEQ ID NO: | Sequence | Content |
| 1 | WGQGTLVTVSS | C-terminal from HCDR3 |
| 2 | WGQGTLVTVSS | C-terminal from HCDR3 |
| 3 | WGQGTLVTVSS | C-terminal from HCDR3 |
| 4 | WGQGTTLTVS | C-terminal from HCDR3 |
| 5 | WGQGTTVTVSSGILGS | C-terminal from HCDR3 |
| 6 | VTVSS | A part of C-terminal from HCDR3 |
| 7 | FGGGTKLEIK | C-terminal from LCDR3 |
| 8 | FGAGTKLELKR | C-terminal from LCDR3 |
| 9 | FGGGTQLTVLS | C-terminal from LCDR3 |
| 10 | KLEIK | A part of C-terminal from LCDR3 |
| 11 | LELKR | A part of C-terminal from LCDR3 |
| 12 | LTVLS | A part of C-terminal from LCDR3 |
| 13 | GGGGS | A part of linker |
| 14 | GGGGSG | A part of linker |
| 15 | SSADD | A part of linker |
| 16 | SSADDA | A part of linker |
| 17 | SSADDAK | A part of linker |
| 18 | SSADDAKK | A part of linker |
| 19 | VTVSSSSADDAKK | An antigen peptide |
| 20 | VTVSSGGGGSG | An antigen peptide |
| 21 | GGVTVSSSSADDAKKC | An antigen peptide |
| 22 | GGVTVSSGGGGSGC | An antigen peptide |
| 23 | LEIKSSADDAKK | An antigen peptide |
| 24 | LEIKGGGGSG | An antigen peptide |
| 25 | GGGGSGGGGS | An example of linkers |
| 26 | GGGGSGGGGSGGGGS | An example of linkers |
| 27 | GGGGSGGGGSGGGGSGGGGS | An example of linkers |

**[Table 11-2]**

| | | |
|---|---|---|
| 28 | GGGGSGGGGSGGGGSGGGGSGGGGS | An example of linkers |
| 29 | SSADDAKKDAAKKDDAKKDDAKKDG | An example of linkers |
| 30 | QVTLKESGPA | A part of N-terminal from HCDR1 |
| 31 | QVQLVQSGAE | A part of N-terminal from HCDR1 |
| 32 | EVQLVQSGAE | A part of N-terminal from HCDR1 |
| 33 | SQVQLKESGP | A part of N-terminal from HCDR1 |
| 34 | QVQLQQSGPG | A part of N-terminal from HCDR1 |
| 35 | DIVMTQSPLS | A part of N-terminal from LCDR1 |
| 36 | DIVMTQSPDS | A part of N-terminal from LCDR1 |
| 37 | DIQLTQSPSS | A part of N-terminal from LCDR1 |
| 38 | DIQMTQSPKF | A part of N-terminal from LCDR1 |
| 39 | QPVLTQSSSL | A part of N-terminal from LCDR1 |
| 40 | SGGGGS | A c-terminal part of a linker |
| 41 | AKKDG | A c-terminal part of a linker |
| 42 | DAKKDG | A c-terminal part of a linker |
| 43 | DDAKKDG | A c-terminal part of a linker |
| 44 | KDDAKKDG | A c-terminal part of a linker |
| 45 | DXXXT | A part of N-terminal from LCDR1 |
| 46 | DIVMT | A part of N-terminal from LCDR1 |
| 47 | DIQLT | A part of N-terminal from LCDR1 |
| 48 | DIQMT | A part of N-terminal from LCDR1 |

**[Table 11-3]**

| | | |
|---|---|---|
| 49 | QPVLT | A part of N-terminal from LCDR1 |
| 50 | KDDAKKDGQVTLK | An antigen peptide |
| 51 | KDDAKKDGQVQLV | An antigen peptide |
| 52 | KDDAKKDGEVQLV | An antigen peptide |
| 53 | KDDAKKDGSQVQL | An antigen peptide |
| 54 | KDDAKKDGQVQLQ | An antigen peptide |
| 55 | SGGGGSQVTLK | An antigen peptide |
| 56 | SGGGGSQVQLV | An antigen peptide |
| 57 | SGGGGSEVQLV | An antigen peptide |
| 58 | SGGGGSSQVQL | An antigen peptide |
| 59 | SGGGGSQVQLQ | An antigen peptide |
| 60 | GGKDDAKKDGQVTLKC | An antigen peptide |
| 61 | GGKDDAKKDGQVQLVC | An antigen peptide |
| 62 | GGKDDAKKDGEVQLVC | An antigen peptide |
| 63 | GGKDDAKKDGSQVQLC | An antigen peptide |
| 64 | GGKDDAKKDGQVQLQC | An antigen peptide |
| 65 | GGSGGGGSQVTLKC | An antigen peptide |
| 66 | GGSGGGGSQVQLVC | An antigen peptide |
| 67 | GGSGGGGSEVQLVC | An antigen peptide |
| 68 | GGSGGGGSSQVQLC | An antigen peptide |
| 69 | GGSGGGGSQVQLQC | An antigen peptide |
| 70 | GGLEIKSSADDAKKC | An antigen peptide |
| 71 | GGLEIKGGGGSGC | An antigen peptide |
| 72 | FGGGTKLEIK | C-terminal ten amino acids in the first region in the CAR in the Examples |
| 73 | FGGGTKVEIK | |
| 74 | FGGGTKLEIK | |
| 75 | GQGTTVTVSS | |
| 76 | GQGTLVTVSA | |
| 77 | WGQGTTLTVS | |
| 78 | GQGTLVTVSS | |
| 79 | TLTVS | A part of C-terminal from HCDR3 |

**[Table 11-3]**

| | | |
|---|---|---|
| 80 | GILGS | A part of C-terminal from HCDR3 |
| 81 | FGGGTKVEIK | C-terminal from LCDR3 |
| 82 | KVEIK | A part of C-terminal from LCDR3 |
| 83 | KDDAKKDGDIVMT | An antigen peptide |
| 84 | KDDAKKDGDIQLT | An antigen peptide |
| 85 | KDDAKKDGDIQMT | An antigen peptide |
| 86 | KDDAKKDGQPVLT | An antigen peptide |
| 87 | SGGGGSDIVMT | An antigen peptide |
| 88 | SGGGGSDIQLT | An antigen peptide |
| 89 | SGGGGSDIQMT | An antigen peptide |
| 90 | SGGGGSQPVLT | An antigen peptide |
| 91 | EVKLVESGGGLVQPGRSLKLSCAASGFN FNDYWMGWVRQAPGKGLEWIGEIKKD SSIINYIPSLKDKFTISRDNAQNTLYLQMS KLGSEDTAIYYCARGVEYYGYHYWGQG TLVTVSS | 2D12-B12, VH |
| 92 | GFNFNDYWMG | 2D12-B12, HCDR1 |
| 93 | EIKKDSSIINYIPSLKD | 2D12-B12, HCDR2 |
| 94 | GVEYYGYHY | 2D12-B12, HCDR3 |
| 95 | DIVMTQSPSSLAVSAGETVTINCKSSQSL LYSGNQKNYLAWYQQKPGQSPKLLIYW ASTRHSGVPARFIGSGSGSDFTLTISSVQ AEDLAIYCCQQYYDIPYTFGAGTKLELK | 2D12-B12, VL |
| 96 | KSSQSLLYSGNQKNYLA | 2D12-B12, LCDR1 |
| 97 | WASTRHS | 2D12-B12, LCDR2 |
| 98 | QQYYDIPYT | 2D12-B12, LCDR3 |
| 99 | QVQLQQSGAALVKPGASVKMSCKASGY TFTDCWVSWVKQSHGKSLEWIGEIHPN SGTTNFNEKFKGKATLTVDKSTSTAYM ELSRLTSEDSAIYYCTRAYGGYPFDYWG QGVMVTVSS | 3A4-A10-7, VH |
| 100 | GYTFTDCWVS | 3A4-A10-7, HCDR1 |
| 101 | EIHPNSGTTNFNEKFKG | 3A4-A10-7, HCDR2 |

**[Table 11-4]**

| | | |
|---|---|---|
| 102 | AYGGYPFDY | 3A4-A10-7, HCDR3 |
| 103 | GAPFAFLHHLTGSCADLVLTQPKSVSTS LESTVKLSCKLNSGNIGRYYMHWYQQHE GRSPTNMIYRDDKRPDGVPDRFSGSIDS SSNSAFLTINNVQTEDEAIYFCHSYDSSI NIFGGGTKLTVLGQPK | 3A4-A10-7, VL |
| 104 | KLNSGNIGRYYMH | 3A4-A10-7, LCDR1 |
| 105 | RDDKRPD | 3A4-A10-7, LCDR2 |
| 106 | HSYDSSINI | 3A4-A10-7, LCDR3 |
| 107 | FGGGTKVEIK | C-terminal from CDR3 in the First Region |
| 108 | FGGGTKLEIK | |
| 109 | GQGTLVTVSS | |
| 110 | GAGTTVTVSS | |
| 111 | GQGTLVTVSA | |
| 112 | FGGGTKVEIK | |
| 113 | FGGGTKLEIK | |
| 114 | FGGGTKLEIK | |
| 115 | FGGGTKLEIK | |
| 116 | FGGGTKVEIK | |
| 117 | GQGTLVTVSS | |
| 118 | GQGTTVTVSSGILGS | |
| 119 | GQGTLVTVSS | |
| 120 | VTVSA | A part of C-terminal from HCDR3 |

**[Table 11-5]**

| | | |
|---|---|---|
| 121 | QVQMKESGPGLVQPSETLSLTCTVSGFSL TSYGVSWVRQPSGKGPEWMGRLWNDGD TAYNSALKSRLSISRDTSKNQVFLKMNSLQ TDDTGTYYCTTMNGYKDYWGQGVMVT VSS | 3B7-C1 VH amino acid sequence |
| 122 | AWTSLLLPLLSLYAGSVTSYELIQPPSASVT LGNTVSLTCVGDELPKRYAYWYQQKPDQ SIVRVIYEDSKRPSGISDRFSGSSSGTTATLT IRDAQAEDEADYYCHSTYSDDKLRIFGGG TKLTVLG | 3E7-C1 VL amino acid sequence |
| 123 | GFSLTSYG | HCDR1 of 3E7-C1 predicted by IMGT |
| 124 | LWNDGDT | HCDR2 of 3E7-C1 predicted by IMGT |
| 125 | TTMNGYKDY | HCDR3 of 3E7-C1 predicted by IMGT |
| 126 | ELPKRY | LCDR1 of 3E7-C1 predicted by IMGT |
| 127 | EDS | LCDR2 of 3E7-C1 predicted by IMGT |
| 128 | HSTYSDDKLRI | LCDR3 of 3E7-C1 predicted by IMGT |
| 129 | TSYGVS | HCDR1 of 3E7-C1 predicted by Kabat |
| 130 | RLWNDGDTAYNSALKS | HCDR2 of 3E7-C1 predicted by Kabat |
| 131 | MNGYKDY | HCDR3 of 3E7-C1 predicted by Kabat |
| 132 | VGDELPKRYAY | LCDR1 of 3E7-C1 predicted by Kabat |
| 133 | EDSKRPS | LCDR2 of 3E7-C1 predicted by Kabat |
| 134 | HSTYSDDKLRI | LCDR3 of 3E7-C1 predicted by Kabat |
| 135 | GFSLTSY | HCDR1 of 3E7-C1 predicted by Chothia |
| 136 | WNDGD | HCDR2 of 3E7-C1 predicted by Chothia |
| 137 | MNGYKDY | HCDR3 of 3B7-C1 predicted by Chothia |
| 138 | VGDELPKRYAY | LCDR1 of 3E7-C1 predicted by Chothia |
| 139 | EDSKRPS | LCDR2 of 3E7-C1 predicted by Chothia |
| 140 | HSTYSDDKLRI | LCDR3 of 3E7-C1 predicted by Chothia |
| 141 | TSYGVS | HCDR1 of 3E7-C1 predicted by Contact |
| 142 | WMGRLWNDGDTA | HCDR2 of 3E7-C1 predicted by Contact |
| 143 | TTMNGYKD | HCDR3 of 3E7-C1 predicted by Contact |
| 144 | KRYAYWY | LCDR1 of 3E7-C1 predicted by Contact |
| 145 | RVIYEDSKRP | LCDR2 of 3E7-C1 predicted by Contact |
| 146 | HSTYSDDKLR | LCDR3 of 3E7-C1 predicted by Contact |
| 147 | GFSLTSYGVS | HCDR1 of 3E7-C1 predicted by AbM |
| 148 | RLWNDGDTA | HCDR2 of 3E7-C1 predicted by AbM |
| 149 | MNGYKDY | HCDR3 of 3E7-C1 predicted by AbM |
| 150 | VGDELPKRYAY | LCDR1 of 3E7-C1 predicted by AbM |
| 151 | EDSKRPS | LCDR2 of 3E7-C1 predicted by AbM |
| 152 | HSTYSDDKLRI | LCDR3 of 3E7-C1 predicted by AbM |
| 153 | QMQLKESGPGLVQPSETLSLTCTVSGF SLTSYSVSWVRQPSGKGPEWMGRMYY DGDTAYNSALKSRLSISRDTSKNQVFL KMNSLQTDDTGTYYCTRSIGAEDYW GQGVMVTVSS | 4B9-B11 VH amino acid sequence |
| 154 | AWTSLLLPLLSLYAGSVTSYELIQPPSA SVTLGNTVSLTCVGDELPKRYAYWYQ QKPDQSIVRVIYEDSKRPSGISDRFSGS SSGTTATLTIRDAQNEDEADYYCHSTY SDDKLRIFGGGTKLTVLG | 4B9-B11 VL amino acid sequence |
| 155 | GFSLTSYS | HCDR1 of 4B9-B11 predicted by IMGT |
| 156 | MYYDGDT | HCDR2 of 4B9-B11 predicted by IMGT |
| 157 | TRSIGAEDY | HCDR3 of 4B9-B11 predicted by IMGT |
| 158 | ELPKRY | LCDR1 of 4B9-B11 predicted by IMGT |
| 159 | EDS | LCDR2 of 4B9-B11 predicted by IMGT |
| 160 | HSTYSDDKLRI | LCDR3 of 4B9-B11 predicted by IMGT |
| 161 | TSYSVS | HCDR1 of 4B9-B11 predicted by Kabat |
| 162 | RMYYDGDTAYNSALKS | HCDR2 of 4B9-B11 predicted by Kabat |
| 163 | SIGAEDY | HCDR3 of 4B9-B11 predicted by Kabat |
| 164 | VGDELPKRYAY | LCDR1 of 4B9-B11 predicted by Kabat |
| 165 | EDSKRPS | LCDR2 of 4B9-B11 predicted by Kabat |
| 166 | HSTYSDDKLRI | LCDR3 of 4B9-B11 predicted by Kabat |
| 167 | GFSLTSY | HCDR1 of 4B9-B11 predicted by Chothia |
| 168 | YYDGD | HCDR2 of 4B9-B11 predicted by Chothia |
| 169 | SIGAEDY | HCDR3 of 4B9-B11 predicted by Chothia |
| 170 | VGDELPKRYAY | LCDR1 of 4B9-B11 predicted by Chothia |
| 171 | EDSKRPS | LCDR2 of 4B9-B11 predicted by Chothia |
| 172 | HSTYSDDKLRI | LCDR3 of 4B9-B11 predicted by Chothia |
| 173 | TSYSVS | HCDR1 of 4B9-B11 predicted by Contact |
| 174 | WMGRMYYDGDTA | HCDR2 of 4B9-B11 predicted by Contact |
| 175 | TRSIGAED | HCDR3 of 4B9-B11 predicted by Contact |
| 176 | KRYAYWY | LCDR1 of 4B9-B11 predicted by Contact |
| 177 | RVIYEDSKRP | LCDR2 of 4B9-B11 predicted by Contact |
| 178 | HSTYSDDKLR | LCDR3 of 4B9-B11 predicted by Contact |
| 179 | GFSLTSYS | HCDR1 of 4B9-B11 predicted by AbM |
| 180 | RMYYDGDTA | HCDR2 of 4B9-B11 predicted by AbM |
| 181 | SIGAEDY | HCDR3 of 4B9-B11 predicted by AbM |
| 182 | VGDELPKRYAY | LCDR1 of 4B9-B11 predicted by AbM |
| 183 | EDSKRPS | LCDR2 of 4B9-B11 predicted by AbM |
| 184 | HSTYSDDKLRI | LCDR3 of 4B9-B11 predicted by AbM |
| 185 | TSY | HCDR1 of 3E7-C1 consensus |
| 186 | WNDGD | HCDR2 of 3E7-C1 consensus |
| 187 | MNGYKD | HCDR3 of 3E7-C1 consensus |
| 188 | KRY | LCDR1 of 3E7-C1 consensus |
| 189 | EDS | LCDR2 of 3E7-C1 consensus |
| 190 | HSTYSDDKLR | LCDR3 of 3E7-C1 consensus |
| 191 | TSY | HCDR1 of 4B9-B11 consensus |
| 192 | YYDGD | HCDR2 of 4B9-B11 consensus |
| 193 | SIGAED | HCDR3 of 4B9-B11 consensus |
| 194 | KRY | LCDR1 of 4B9-B11 consensus |
| 195 | EDS | LCDR2 of 4B9-B11 consensus |
| 196 | HSTYSDDKLR | LCDR3 of 4B9-B11 consensus |

## Claims

1. An antibody that binds to an scFv comprising a first region, a linker, and a second region, wherein the first region, the linker, and the second region in the scFv are arranged in this order in the direction from the N terminal to the C terminal,
the first region and second region are a heavy-chain variable region of an antibody and a light-chain variable region of the antibody, respectively, or a light-chain variable region of an antibody and a heavy-chain variable region of the antibody, respectively, and
the antibody is an antibody satisfying at least one of the following (i) to (vi):
(i) the antibody binds to an isolated peptide having an amino acid sequence of (i-a) a region present within the first region and having 1 to 10 amino acids from the C terminal of the first region, and (i-b) a partial region of a linker continuous with the region and having 1 to 10 amino acids from the N terminal of the linker, the isolated peptide having 13 to 20 amino acid length,
(ii) the antibody binds to an isolated peptide having an amino acid sequence of (ii-a) a partial region of a linker continuous with the second region and having 1 to 10 amino acids from the C terminal of the linker, and (ii-b) a region present within the second region and having 1 to 10 amino acids from the N terminal of the second region, the isolated peptide having 13 to 20 amino acid length,
(iii) the antibody binds to an isolated peptide having an amino acid sequence set forth in any one of SEQ ID NOs: 19, 20, 23 and 24,
(iv) the antibody binds to an isolated peptide having an amino acid sequence set forth in any one of SEQ ID NOs: 19 to 24 and 50 to 71,
(v) the antibody binds to (v-a) a region present within the first region and having 1 to 10 amino acids from the C terminal of the first region, and/or (v-b) a partial region of a linker continuous with the region and having 1 to 10 amino acids from the N terminal of the linker, and
(vi) the antibody binds to (vi-a) a partial region of a linker continuous with the second region and having 1 to 10 amino acids from the C terminal of the linker, and/or (vi-b) a region present within the second region and having 1 to 10 amino acids from the N terminal of the second region.

2. The antibody according to claim 1, wherein (i) the antibody binds to an isolated peptide having an amino acid sequence of (i-a) a region present within the first region and having 1 to 10 amino acids from the C terminal of the first region, and (i-b) a partial region of a linker continuous with the region and having 1 to 10 amino acids from the N terminal of the linker, the isolated peptide having a 13 to 20 amino acid length.

3. The antibody according to claim 1, wherein (ii) the antibody binds to an isolated peptide having an amino acid sequence of (ii-a) a partial region of a linker continuous with the second region and having 1 to 10 amino acids from the C terminal of the linker, and (ii-b) a region present within the second region and having 1 to 10 amino acids from the N terminal of the second region, the isolated peptide having a 13 to 20 amino acid length.

4. The antibody according to claim 1, wherein (iii) the antibody binds to an isolated peptide having an amino acid sequence set forth in any one of SEQ ID NOs: 19, 20, 23 and 24.

5. The antibody according to claim 1, wherein (iv) the antibody binds to an isolated peptide having an amino acid sequence set forth in any one of SEQ ID NOs: 19 to 24 and 50 to 71.

6. The antibody according to claim 1, wherein (v) the antibody binds to (v-a) a region present within the first region and having 1 to 10 amino acids from the C terminal of the first region, and/or (v-b) a partial region of a linker continuous with the region and having 1 to 10 amino acids from the N terminal of the linker.

7. The antibody according to claim 1, wherein (vi) the antibody binds to (vi-a) a partial region of a linker continuous with the second region and having 1 to 10 amino acids from the C terminal of the linker, and/or (vi-b) a region present within the second region and having 1 to 10 amino acids from the N terminal of the second region.

8. The antibody according to any one of claims 1 to 7, wherein (vii) the antibody exhibits no significant binding affinity for an scFv in which the order of the heavy-chain variable region and the light-chain variable region is interchanged.

9. The antibody according to any one of claims 1 to 7, wherein (viii) the antibody exhibits significant binding affinity even for an scFv in which the order of the heavy-chain variable region and the light-chain variable region is interchanged.

10. The antibody according to any one of claims 1 to 9, wherein the antibody binds to a chimeric antigen receptor (CAR) comprising the scFv.

11. The antibody according to any one of claims 1 to 10, wherein the antibody binds to a cell expressing the chimeric antigen receptor (CAR) comprising the scFv.

12. A method for detecting a cell expressing a chimeric antigen receptor (CAR) comprising an scFv, comprising bringing the cell into contact with the antibody according to claim 11.

13. A kit or test agent for detecting a cell expressing a chimeric antigen receptor (CAR) comprising an scFv, wherein the kit or test agent comprises the antibody according to claim 11.

14. A method for targeting a cell expressing a chimeric antigen receptor (CAR) comprising an scFv in a subject, comprising
administering the cell expressing the chimeric antigen receptor (CAR) comprising an scFv to the subject and
administering the antibody according to claim 11 to the subject.

15. An antibody formulation for use in targeting a cell expressing a chimeric antigen receptor (CAR) comprising an scFv in a subject administered with the cell expressing a chimeric antigen receptor (CAR) comprising an scFv, wherein the antibody formulation comprises the antibody according to claim 11.

16. A method for obtaining an antibody that binds to an scFv, wherein
the scFv comprises a first region, a linker, and a second region, wherein the first region, the linker, and the second region in the scFv are arranged in this order in the direction from the N terminal to the C terminal, and the first region and second region are a heavy-chain variable region of an antibody and a light-chain variable region of an antibody, respectively, or a light-chain variable region of an antibody and a heavy-chain variable region of an antibody, respectively,
the method comprising selecting or identifying, from a group of antibodies, an antibody that binds to an scFv and satisfying at least one of (i) to (vi):
(i) the antibody binds to an isolated peptide having an amino acid sequence of a region present within the first region and having 1 to 10 amino acids from the C terminal of the first region, and a partial region of a linker continuous with the region and having 1 to 10 amino acids from the N terminal of the linker, the isolated peptide having 13 to 20 amino acid length,
(ii) the antibody binds to an isolated peptide having an amino acid sequence of a partial region of a linker continuous with the second region and having 1 to 10 amino acids from the C terminal of the linker, and a region present within the second region and having 1 to 10 amino acids from the N terminal of the second region, the isolated peptide having 13 to 20 amino acid length,
(iii) the antibody binds to an isolated peptide having an amino acid sequence set forth in any one of SEQ ID NOs: 19, 20, 23 and 24,
(iv) the antibody binds to an isolated peptide having an amino acid sequence set forth in any one of SEQ ID NOs: 19 to 24 and 50 to 71,
(v) the antibody binds to a region present within the first region and having 1 to 10 amino acids from the C terminal of the first region and a partial region of a linker continuous with the region and having 1 to 10 amino acids from the N terminal of the linker, and
(vi) the antibody binds to a partial region of a linker continuous with the second region and having 1 to 10 amino acids from the C terminal of the linker, and a region present within the second region and having 1 to 10 amino acids from the N terminal of the second region.

17. The method according to claim 16, wherein (vii) the antibody exhibits no significant binding affinity for an scFv in which the order of the heavy-chain variable region and the light-chain variable region is interchanged.

18. The method according to claim 16, wherein (viii) the antibody exhibits significant binding affinity even for an scFv in which the order of the heavy-chain variable region and the light-chain variable region is interchanged.

19. The method according to any one of claims 16 to 18, further comprising allowing an antibody-producing cell to produce an antibody comprising amino acid sequences of heavy-chain CDR1 to 3 and light-chain CDR1 to 3 of the antibody screened.

20. An antibody that binds to an scFv, wherein:
(i) the antibody comprises a heavy-chain variable region comprising heavy-chain CDR1 to 3 of a heavy-chain variable region having an amino acid sequence set forth in SEQ ID NO: 91, as heavy-chain CDR1 to 3 thereof, respectively, and a light-chain variable region having light-chain CDR1 to 3 of a light-chain variable region having an amino acid sequence set forth in SEQ ID NO: 95 as light-chain CDR1 to 3 thereof, respectively;
(ii) the antibody comprises a heavy-chain variable region comprising heavy-chain CDR1 having an amino acid sequence set forth in SEQ ID NO: 92, heavy-chain CDR2 having an amino acid sequence set forth in SEQ ID NO: 93, and heavy-chain CDR3 having an amino acid sequence set forth in SEQ ID NO: 94; and a light-chain variable region comprising light-chain CDR1 having an amino acid sequence set forth in SEQ ID NO: 96, light-chain CDR2 having an amino acid sequence set forth in SEQ ID NO: 97, and light-chain CDR3 having an amino acid sequence set forth in SEQ ID NO: 98;
(iii) the antibody comprises a heavy-chain variable region comprising heavy-chain CDR1 to 3 in a heavy-chain variable region having an amino acid sequence set forth in SEQ ID NO: 99, as heavy-chain CDR1 to 3 thereof, respectively, and a light-chain variable region having light-chain CDR1 to 3 in a light-chain variable region having an amino acid sequence set forth in SEQ ID NO: 100 as light-chain CDR1 to 3 thereof, respectively;
(iv) the antibody comprises a heavy-chain variable region comprising heavy-chain CDR1 having an amino acid sequence set forth in SEQ ID NO: 100, heavy-chain CDR2 having an amino acid sequence set forth in SEQ ID NO: 101, and heavy-chain CDR3 having an amino acid sequence set forth in SEQ ID NO: 102; and a light-chain variable region comprising light-chain CDR1 having an amino acid sequence set forth in SEQ ID NO: 104, light-chain CDR2 having an amino acid sequence set forth in SEQ ID NO: 105, and light-chain CDR3 having an amino acid sequence set forth in SEQ ID NO: 106;
(v) the antibody comprises a heavy-chain variable region comprising heavy-chain CDR1 to 3 in a heavy-chain variable region having an amino acid sequence set forth in SEQ ID NO: 121, as heavy-chain CDR1 to 3 thereof, respectively, and a light-chain variable region having light-chain CDR1 to 3 in a light-chain variable region having an amino acid sequence set forth in SEQ ID NO: 122 as light-chain CDR1 to 3 thereof, respectively;
(vi) the antibody comprises a heavy-chain variable region comprising heavy-chain CDR1 to 3 in a heavy-chain variable region having an amino acid sequence set forth in SEQ ID NO: 153, as heavy-chain CDR1 to 3 thereof, respectively, and a light-chain variable region having light-chain CDR1 to 3 in a light-chain variable region having an amino acid sequence set forth in SEQ ID NO: 154 as light-chain CDR1 to 3 thereof, respectively;
(vii) the antibody comprises a heavy-chain variable region having an amino acid sequence set forth in SEQ ID NO: 91 and a light-chain variable region having an amino acid sequence set forth in SEQ ID NO: 95;
(viii) the antibody comprises a heavy-chain variable region having an amino acid sequence set forth in SEQ ID NO: 99 and a light-chain variable region having an amino acid sequence set forth in SEQ ID NO: 103;
(ix) the antibody comprises a heavy-chain variable region having an amino acid sequence set forth in SEQ ID NO: 121 and a light-chain variable region having an amino acid sequence set forth in SEQ ID NO: 122;
(x) the antibody comprises a heavy-chain variable region having an amino acid sequence set forth in SEQ ID NO: 153 and a light-chain variable region having an amino acid sequence set forth in SEQ ID NO: 154;
(xi) the antibody competes with an antibody according to any one of (vii), (viii), (ix), and (x) for binding to an scFv;
(xii) the antibody is any one of the antibodies mentioned above, which does not substantially bind to a naturally occurring (in vivo) immunoglobulin, or
(xiii) the antibody has an identity of 90% or more with the amino acid sequence of the antibody according to any one of the antibodies mentioned above.

21. A method for obtaining an antibody that binds to an scFv, comprising selecting or identifying, from a group of antibodies, an antibody that binds to an scFv and satisfying any one of
(i) the antibody comprising a heavy-chain variable region comprising heavy-chain CDR1 to 3 of a heavy-chain variable region having an amino acid sequence set forth in SEQ ID NO: 91, as heavy-chain CDR1 to 3 thereof, respectively, and a light-chain variable region having light-chain CDR1 to 3 of a light-chain variable region having an amino acid sequence set forth in SEQ ID NO: 95 as light-chain CDR1 to 3 thereof, respectively;
(ii) the antibody comprising a heavy-chain variable region comprising heavy-chain CDR1 having an amino acid sequence set forth in SEQ ID NO: 92, heavy-chain CDR2 having an amino acid sequence set forth in SEQ ID NO: 93, and heavy-chain CDR3 having an amino acid sequence set forth in SEQ ID NO: 94, and a light-chain variable region comprising light-chain CDR1 having an amino acid sequence set forth in SEQ ID NO: 96, light-chain CDR2 having an amino acid sequence set forth in SEQ ID NO: 97, and light-chain CDR3 having an amino acid sequence set forth in SEQ ID NO: 98;
(iii) the antibody comprising a heavy-chain variable region comprising heavy-chain CDR1 to 3 in a heavy-chain variable region having an amino acid sequence set forth in SEQ ID NO: 99, as heavy-chain CDR1 to 3 thereof, respectively, and a light-chain variable region having light-chain CDR1 to 3 in a light-chain variable region having an amino acid sequence set forth in SEQ ID NO: 100 as light-chain CDR1 to 3 thereof, respectively;
(iv) the antibody comprising a heavy-chain variable region comprising heavy-chain CDR1 having an amino acid sequence set forth in SEQ ID NO: 100, heavy-chain CDR2 having an amino acid sequence set forth in SEQ ID NO: 101, and heavy-chain CDR3 having an amino acid sequence set forth in SEQ ID NO: 102; and a light-chain variable region comprising light-chain CDR1 having an amino acid sequence set forth in SEQ ID NO: 104, light-chain CDR2 having an amino acid sequence set forth in SEQ ID NO: 105, and light-chain CDR3 having an amino acid sequence set forth in SEQ ID NO: 106;
(v) the antibody comprising a heavy-chain variable region comprising heavy-chain CDR1 to 3 in a heavy-chain variable region having an amino acid sequence set forth in SEQ ID NO: 121, as heavy-chain CDR1 to 3 thereof, respectively, and a light-chain variable region having light-chain CDR1 to 3 in a light-chain variable region having an amino acid sequence set forth in SEQ ID NO: 122 as light-chain CDR1 to 3 thereof, respectively;
(vi) the antibody comprising a heavy-chain variable region comprising heavy-chain CDR1 to 3 in a heavy-chain variable region having an amino acid sequence set forth in SEQ ID NO: 153, as heavy-chain CDR1 to 3 thereof, respectively, and a light-chain variable region having light-chain CDR1 to 3 in a light-chain variable region having an amino acid sequence set forth in SEQ ID NO: 154 as light-chain CDR1 to 3 thereof;
(vii) the antibody comprising a heavy-chain variable region having an amino acid sequence set forth in SEQ ID NO: 91 and a light-chain variable region having an amino acid sequence set forth in SEQ ID NO: 95;
(viii) the antibody comprising a heavy-chain variable region having an amino acid sequence set forth in SEQ ID NO: 99 and a light-chain variable region having an amino acid sequence set forth in SEQ ID NO: 103;
(ix) the antibody comprising a heavy-chain variable region having an amino acid sequence set forth in SEQ ID NO: 121 and a light-chain variable region having an amino acid sequence set forth in SEQ ID NO: 122;
(x) the antibody comprising a heavy-chain variable region having an amino acid sequence set forth in SEQ ID NO: 153 and a light-chain variable region having an amino acid sequence set forth in SEQ ID NO: 154;
(xi) the antibody competing with an antibody according to any one of (vii), (viii), (ix), and (x) for binding to an scFv;
(xii) the antibody being any one of the antibodies mentioned above, which does not substantially bind to a naturally occurring (in vivo) immunoglobulin; or
(xiii) the antibody having an identity of 90% or more with the amino acid sequence of the antibody according to any one of the antibodies mentioned above.
